# EUROPEAN PATENT APPLICATION

(11) **EP 4 737 484 A1**
(43) Date of publication of application: **06.05.2026**
(21) Application number: 24831053.4
(22) Date of filing: 28.06.2024
(51) Int. Cl.: C07K 19/00, C07K 16/24, A61K 39/395, A61P 35/00

(54) **BISPECIFIC ANTIBODY TARGETING PD-L1 AND IL-1 AND USE THEREOF**

(30) Priority: 28.06.2023 CN 202310779071
(71) Applicant: Phil Rivers Technology Co., Ltd, Beijing 100190 (CN)
(72) Inventor: ZHANG, Tingyan, Beijing 100190 (CN); YANG, Jianfei, Beijing 100190 (CN); NIU, Gang, Beijing 100190 (CN); ZHANG, Chunli, Beijing 100190 (CN); ZHANG, Chunming, Beijing 100190 (CN)
(74) Representative: Schüssler, Andrea
(86) International application number: PCT/CN2024/102666
(87) International publication number: WO 2025/002429

(57) **Abstract**

Provided is a bispecific antibody comprising a first moiety targeting PD-L1 and a second moiety targeting IL-1, wherein the first moiety comprises an anti-PD-L1 antibody or an antigen-binding fragment thereof, and the second moiety comprises an extracellular binding domain of an IL-1 receptor (IL-1R) or a variant thereof or an anti-IL-1β antibody or an antigen-binding fragment thereof. Further provided is the use of the bispecific antibody in the treatment of tumors.

## Description

### Technical Field

The present invention relates to a bispecific antibody, especially a bispecific antibody comprising a first moiety targeting PD-L1 and a second moiety targeting IL-1. The present disclosure also relates to use of the bispecific antibody in the treatment of a tumor.

### Background

The mechanism of action is to target both PD-L1 on a surface of a tumor cell and IL-1R1 or IL-1β in a tumor microenvironment, so as to achieve a better therapeutic effect than a single drug or a combination of single drugs.

Studies have shown that PD-L1 is highly expressed on surfaces of a variety of tumor cells. The PD-L1 on the surface of a tumor cell, after binding to PD-1 on the surface of a T cell, inhibits killing of the tumor cell by the T cell and reduces the proliferation of a CD8⁺ T cell while reducing the apoptosis of a Treg. An antibody targeting PD-L1 can inhibit the binding of PD-L1 to PD-1, thereby restoring the killing effect of the T cell on the tumor, which shows a significant therapeutic effect in treating patients with various types of tumors such as malignant melanoma, non-small cell lung cancer, renal cell carcinoma, bladder cancer, or gastric cancer.

L-1β is a member of the IL-1 cytokine family. After binding to IL-1R1, IL-1α or IL-1β binds to an IL-1R accessory protein (IL-1RAcP) to form a complex. The complex can activate an IL-1R associated kinase (IRAK) and further, through NF-κB and other transcription factors, activates a nuclear gene finally. Studies have shown that IL-1β is upregulated in tumor microenvironments (TME) of a variety of cancers, including breast cancer, gastric cancer, pancreatic cancer, and lung cancer. It can change the components of the TME by recruiting an immunosuppressive cell such as an MDSC, a tumor-associated macrophage (TAM), a tumor-associated neutrophil (TAN), and Th17, which are themselves sources of IL-1β. In addition, recent studies have shown that a tumor itself is also a source of IL-1β, as it can establish a self-stimulating cycle to sustain the synthesis and release of IL-1β. It has also been demonstrated that a tumor-derived IL-1β can promote the expression of an angiogenic factor such as VEGF. In summary, a TME-derived IL-1β and a tumor-derived IL-1β can not only promote tumor growth and migration, but also participate in the development of tumor resistance to a drug by inducing anti-apoptotic signaling. It has been demonstrated that an antibody targeting IL-1β can inhibit the growth and migration of tumors, improve the infiltration of a CD8⁺ T cell into a tumor tissue, and reduce the immunosuppression.

The latest preclinical and clinical data have shown that the combination of an immune checkpoint inhibitor with a drug which negatively regulates IL-1 signaling is a promising strategy for treating a solid tumor. And compared to a combination of single drugs, an antibody fusion protein or a bispecific antibody targeting both PD-L1 and IL-1R1 or both PD-L1 and IL-1β have the advantages of high specificity, strong targeting property, high safety level, convenient administration, and low development cost, and the like.

### Summary

In an aspect, provided herein is a bispecific antibody comprising a first moiety targeting PD-L1 and a second moiety targeting IL-1, wherein the first moiety comprises an anti-PD-L1 antibody or an antigen-binding fragment thereof, and the anti-PD-L1 antibody comprises an HCDR1, an HCDR2, and an HCDR3 from a heavy chain variable region (VH) shown in SEQ ID NO: 17, and an LCDR1, an LCDR2, and an LCDR3 from a light chain variable region (VL) shown in SEQ ID NO: 18.

In some embodiments, the anti-PD-L1 antibody comprises a sequence shown in SEQ ID NO: 17 or an amino acid sequence having at least 90% sequence identity therewith, and a sequence shown in SEQ ID NO: 18 or an amino acid sequence having at least 90% sequence identity therewith.

In some embodiments, a heavy chain constant region of the anti-PD-L1 antibody is from an IgG1, and a light chain constant region of the anti-PD-L1 antibody is from a κ light chain.

In some embodiments, the second moiety comprises an extracellular binding domain of an IL-1 receptor (IL-1R) or a variant thereof.

In some embodiments, the second moiety comprises a sequence shown in SEQ ID NO: 14 or an amino acid sequence having at least 90% sequence identity therewith.

In some embodiments, the number of the second moiety is two, and the second moieties are respectively connected at an N-terminus of the VH of the anti-PD-L1 antibody, an N-terminus of the VL of the anti-PD-L1 antibody, a C-terminus of the heavy chain constant region of the anti-PD-L1 antibody, or a C-terminus of the light chain constant region of the anti-PD-L1 antibody via an identical or different linker sequence.

In some embodiments, the number of the second moiety is one, and the second moiety is connected at an N-terminus of the VH of the anti-PD-L1 antibody, an N-terminus of the VL of the anti-PD-L1 antibody, a C-terminus of the heavy chain constant region of the anti-PD-L1 antibody, or a C-terminus of the light chain constant region of the anti-PD-L1 antibody via a linker sequence.

In some embodiments, the first moiety further has an antigen-binding fragment of the anti-PD-L1 antibody in an Fab format connected via a linker sequence to a C-terminus of the heavy chain constant region of the anti-PD-L1 antibody.

In some embodiments, the second moiety is an anti-IL-1β antibody or an antigen-binding fragment thereof.

In some embodiments, the anti-IL-1β antibody comprises an HCDR1, an HCDR2, and an HCDR3 from a VH shown in SEQ ID NO: 15, and an LCDR1, an LCDR2, and an LCDR3 from a VL shown in SEQ ID NO: 16.

In some embodiments, the anti-IL-1β antibody comprises a sequence shown in SEQ ID NO: 15 or an amino acid sequence having at least 90% sequence identity therewith, and a sequence shown in SEQ ID NO: 16 or an amino acid sequence having at least 90% sequence identity therewith.

In some embodiments, a heavy chain constant region of the anti-IL-1β antibody is from an IgG1, and a light chain constant region of the anti-PD-L1 antibody is from a κ light chain.

In some embodiments, the antigen-binding fragment of the anti-IL-1β antibody is in an Fab or scFv format.

In some embodiments, the heavy chain constant region of the anti-PD-L1 antibody and/or the heavy chain constant region of the anti-IL-1β antibody are/is mutated so that a Knob-hole binding is capable of being formed between the heavy chain constant regions.

In some embodiments, the first moiety and the second moiety are connected via an identical or different linker sequence, and preferably, the linker sequence is selected from (G₄S)₄G, (G₄S)₃G, (G₄S)₄ and (G₄S)₃.

In some embodiments, a heavy chain of the anti-PD-L1 antibody comprises a sequence shown in SEQ ID NO: 9 or an amino acid sequence having at least 90% sequence identity therewith, and a light chain of the anti-PD-L1 antibody comprises a sequence shown in SEQ ID NO: 8 or an amino acid sequence having at least 90% sequence identity therewith.

In some embodiments, a heavy chain of the anti-IL-1β antibody comprises a sequence shown in SEQ ID NO: 7 or an amino acid sequence having at least 90% sequence identity therewith, and a light chain of the anti-IL-1β antibody comprises a sequence shown in SEQ ID NO: 6 or an amino acid sequence having at least 90% sequence identity therewith.

In some embodiments, the antigen-binding fragment of the anti-PD-L1 antibody in an scFv format comprises a sequence shown in SEQ ID NO: 66 or an amino acid sequence having at least 90% sequence identity therewith.

In some embodiments, the antigen-binding fragment of the anti-IL-1β antibody in an scFv format comprises a sequence shown in SEQ ID NO: 64 or 65, or an amino acid sequence having at least 90% sequence identity therewith.

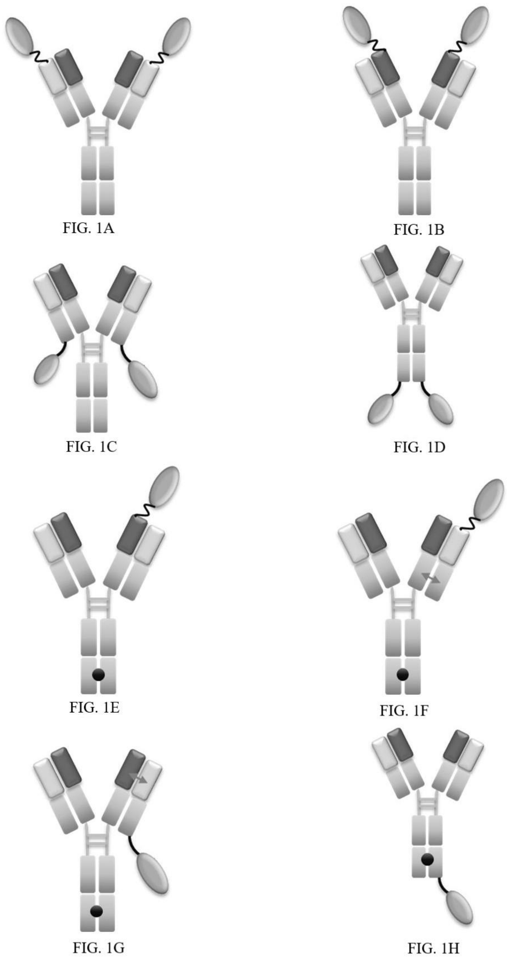

In some embodiments, the bispecific antibody has any of the schematic antibody structures in Figures 1A-1I.

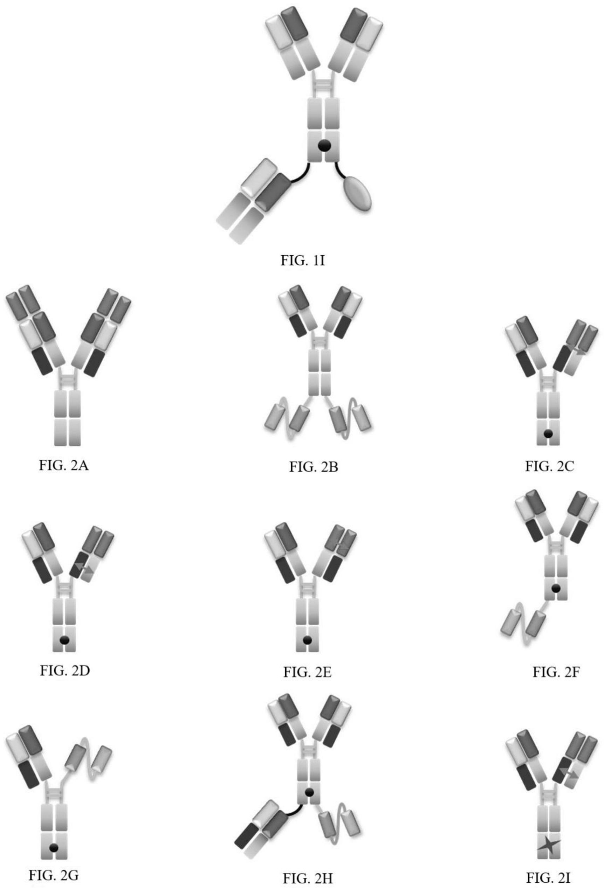

In some embodiments, the bispecific antibody has any of the schematic antibody structures in Figures 2A-2J.

In some embodiments, the extracellular binding domain of the IL-1 receptor (IL-1R) or a variant thereof is at its C-terminus connected to an Fc fragment.

In some embodiments, the heavy chain constant region and/or the Fc fragment comprise(s) an N297A mutation.

In some embodiments, the heavy chain constant region and/or the Fc fragment are/is from a human IgG1.

In some embodiments, the heavy chain constant region comprises a sequence shown in SEQ ID NO: 68, and the Fc fragment comprises a sequence shown in SEQ ID NO: 67.

In some embodiments, the second moiety comprises a sequence shown in SEQ ID NO: 10 or 66.

In some embodiments, the bispecific antibody comprises a polypeptide chain listed in any row of Table 1.

In some embodiments, the bispecific antibody comprises:
1) a polypeptide chain with a sequence shown in SEQ ID NO: 25 and a polypeptide chain with a sequence shown in SEQ ID NO: 26;
2) a polypeptide chain with a sequence shown in SEQ ID NO: 25 and a polypeptide chain with a sequence shown in SEQ ID NO: 30;
3) a polypeptide chain with a sequence shown in SEQ ID NO: 32 and a polypeptide chain with a sequence shown in SEQ ID NO: 28;
4) a polypeptide chain with a sequence shown in SEQ ID NO: 40, a polypeptide chain with a sequence shown in SEQ ID NO: 39, and a polypeptide chain with a sequence shown in SEQ ID NO: 28;
5) a polypeptide chain with a sequence shown in SEQ ID NO: 43 and a polypeptide chain with a sequence shown in SEQ ID NO: 44;
6) a polypeptide chain with a sequence shown in SEQ ID NO: 45 and a polypeptide chain with a sequence shown in SEQ ID NO: 46;
7) a polypeptide chain with a sequence shown in SEQ ID NO: 33, a polypeptide chain with a sequence shown in SEQ ID NO: 28, a polypeptide chain with a sequence shown in SEQ ID NO: 50, and a polypeptide chain with a sequence shown in SEQ ID NO: 51;
8) a polypeptide chain with a sequence shown in SEQ ID NO: 62 and a polypeptide chain with a sequence shown in SEQ ID NO: 63;
9) a polypeptide chain with a sequence shown in SEQ ID NO: 69 and a polypeptide chain with a sequence shown in SEQ ID NO: 30;
10) a polypeptide chain with a sequence shown in SEQ ID NO: 70 and a polypeptide chain with a sequence shown in SEQ ID NO: 28;
11) a polypeptide chain with a sequence shown in SEQ ID NO: 71 and a polypeptide chain with a sequence shown in SEQ ID NO: 46; or
12) a polypeptide chain with a sequence shown in SEQ ID NO: 72 and a polypeptide chain with a sequence shown in SEQ ID NO: 63.

In another aspect, provided herein is a nucleic acid molecule encoding the above bispecific antibody or a polypeptide chain thereof.

In another aspect, provided herein is an expression vector comprising the above nucleic acid molecule.

In another aspect, provided herein is a pharmaceutical composition comprising:
1) the above bispecific antibody, nucleic acid molecule, or expression vector; and
2) a pharmaceutically acceptable carrier.

In another aspect, provided herein is use of the above bispecific antibody, nucleic acid molecule, or expression vector in the manufacture of a medicament for treating a tumor.

In some embodiments, the tumor is selected from malignant melanoma, non-small cell lung cancer, renal cell carcinoma, bladder cancer, gastric cancer, breast cancer, pancreatic cancer, and lung cancer.

In another aspect, provided herein is a method of treating a tumor in a subject, comprising administering to the subject a therapeutically effective amount of the above bispecific antibody, nucleic acid molecule, expression vector, or pharmaceutical composition.

In some embodiments, the tumor is selected from malignant melanoma, non-small cell lung cancer, renal cell carcinoma, bladder cancer, gastric cancer, breast cancer, pancreatic cancer, and lung cancer.

### Description of the Drawings

Figures 1A-1I are schematic diagrams showing the structures of the bispecific antibodies of the present invention including Anakinra.
Figures 2A-2J are schematic diagrams showing the structures of the bispecific antibodies of the present invention including an anti-IL-1β antibody or an antigen-binding fragment thereof.
Figures 3A-3H show the results of the binding affinities of the bispecific antibodies of the present invention to huPD-L1-His.
Figures 4A-4H show the results of the binding affinities of the bispecific antibodies of the present invention to huIL-1R1-His or IL-1β-His.
Figures 5A-5F show the results of the bispecific antibodies of the present invention in blocking the IL-1β/IL-1R1 activity.
Figures 6A-6H show the results of the binding affinities of the bispecific antibodies of the present invention to the huPD-L1-CHO-K cells.
Figures 7A-7I show the results of the bispecific antibodies of the present invention in blocking the PD-1/PD-L1 binding activity.
Figures 8A-8H show the results of the bispecific antibodies of the present invention in blocking the PD-1/PD-L1 signaling pathway.
Figures 9A-9H show the results of the abilities of the bispecific antibodies of the present invention in binding both the huPD-L1 and IL-1R1 or IL-1β.
Figures 10A-10I show the SDS-PAGE results of the bispecific antibodies of the present invention and the reference IPI.
Figures 11A-11AD show the SEC-HPLC results of the bispecific antibodies of the present invention.

### Detailed Description

Unless otherwise stated, all technical or scientific terms used herein have the meaning as commonly understood by one of ordinary skill in the art.

IL-1β and IL-1R1 are most important inflammatory factor and the receptor thereof in a tumor microenvironment, and the blockage of their signaling pathway has been demonstrated to inhibit tumor growth and metastasis, and help remove the immune checkpoint inhibition in the tumor microenvironment. The combination of immune checkpoint inhibitor with the drug which blocks the IL-1 signaling pathway is a novel and promising strategy for treating solid tumors. And compared to the combination of single drugs, the antibody fusion protein or bispecific antibody against two targets have the advantages of strong targeting property, high safety level, convenient administration, and low development cost and the like, and may reduce tumor escape and overcome drug resistance.

The present invention generally described herein will be more readily understood by reference to the following Examples, which are provided in an exemplary manner and are not intended to limit the present invention. In the following Examples, the experimental methods which do not specify the specific condition were carried out in accordance with conventional methods and conditions, or in accordance with the instructions of the commodities.

### Example 1 Preparation of raw materials

### 1.1 Preparation of antigens

The IL-1 used in the present invention was IL-1β, and the receptor as mainly used was IL-1R1. The antigen proteins huIL-1β-Fe, huIL-1β-His, huIL-1R1-Fc, huIL-1R1-His, huPD-L1-Fc and huPD-L1-His are prepared by fusing the human IgG1 Fc segment (SEQ ID NO: 4) or 6×His tag (such as SEQ ID NO: 5) at C-terminuses of the extracellular region of human IL-1β (UNIPROT: P01584, SEQ ID NO: 1), the extracellular region of human IL-1R1 (UNIPROT: P14778, SEQ ID NO: 2), and the extracellular region of human PD-L1 (UNIPROT: Q9NZQ7, SEQ ID NO: 3). After converting the above amino acid sequences into gene sequences, the gene fragments of interest were synthesized by General Biological Technology Co., Ltd. Each fragment of interest was amplified by PCR and then incorporated into a pcDNA3.4 (Invitrogen) expression vector using a homologous recombination-based method. The constructed expression vectors for the various recombinant proteins were respectively transformed into *Escherichia coli* SS320, and cultured overnight at 37°C. Then plasmids were extracted using an endotoxin-free plasmid extraction kit (OMEGA, D6950-01) to obtain various endotoxin-free plasmids for use in eukaryotic expression. Expression was carried out using the obtained expression vectors with the Expi293 Transient Transfection Expression System (ThermoFisher, A14635). See Expi293^{™} Expression System USER GUIDE for the transient transfection methods.

5-7 days after transfection, the supernatants from cell expression were purified based on different tags respectively. For the supernatants with His-tagged proteins, affinity purification was performed using Ni Smart Beads 6FF (Changzhou Smart-Lifesciences Biotechnology Co., Ltd., SA036050), followed by elution of the proteins of interest using imidazole concentration gradient. The various eluted proteins were respectively exchanged into the buffer through an ultrafiltration concentration tube (Millipore, UFC901096). Following SDS-PAGE identification and activity identification, eligible proteins were cryopreserved at -80°C. For supernatants with Fc-tagged proteins, they were filtered by a 0.22 µm filter membrane, and then purified based on affinity using a Protein A/G affinity chromatography column. After purification, the proteins of interest were eluted with 100 mM glycinate (pH 3.0), then concentrated and exchanged. Following SDS-PAGE identification and activity identification, eligible proteins were cryopreserved at -80°C.

### 1.2 Preparation of positive control molecules

The positive control antibody blocking IL-1 activity prepared in the present Example was Canakinumab, and the anti-PD-L1 positive control antibody was Atezolizumab (Genentech). They were synthesized according to the sequences disclosed in the international application WO2001053353A2 and the US patent US8217149B respectively (the light and heavy chains of Canakinumab were SEQ ID NO: 6 and SEQ ID NO: 7 respectively, and the light and heavy chains of Atezolizumab were SEQ ID NO: 8 and SEQ ID NO: 9 respectively). Molecular cloning methods were used to construct a plasmid containing a Canakinumab light chain gene, a plasmid containing a Canakinumab heavy chain gene, a plasmid containing an Atezolizumab light chain gene, and a plasmid containing an Atezolizumab heavy chain gene, respectively. Expression was carried out using the ExpiCHO Transient Transfection System. The resulting supernatants were filtered at 0.22 µm, purified using a Protein A/G affinity purification method, and then eluted with 100 mM glycinate (pH 3.0) to obtain the positive control antibodies Canakinumab and Atezolizumab.

In this Example, the positive control proteins Anakinra-Fc (SEQ ID NO: 10) and Anakinra-Fc (N297A) (SEQ ID NO: 66) were also prepared using the same methods.

### 1.3 Construction of cell lines

### 1.3.1 Construction of huIL-1R1-HEK293 cell line

A recombinant plasmid vector expressing full-length human IL-1R1 (UniProtKB-P14778, SEQ ID NO: 11) was constructed, and introduced into HEK293 cells (ATCC^{®} CRL-1573TM) by electroporation. Flow cytometry (Beckman, CytoFLEX AOO-1-1102) was carried out using the positive control Anakinra-Fc or IL-1β-Fc to screen and identify the cell line highly expressing human IL-1R1, which was named Hu-IL-1R1-FL-HEK293 cell.

### 1.3.2 Construction of IL-1R1-NF-κB-HEK293 cell line

A recombinant plasmid vector expressing full-length human IL-1R1 and an NF-κB luciferase reporter gene plasmid (containing a luciferase reporter gene whose expression was driven by an NF-κB response element) were constructed. The above plasmids were co-introduced into HEK293 cells (ATCC^{®} CRL-1573TM) by electroporation. Puromycin at a final concentration of 2 µg/mL was used to screen a monoclonal cell line, and then flow cytometry (Beckman, CytoFLEX AOO-1-1102) was carried out using the positive control Anakinra-Fc or IL-1β-Fc to screen and identify the cell line highly expressing human IL-1R1. And luciferase detection was used to screen the IL-1R1-NF-κB-HEK293 cell.

### 1.3.3 Construction of huPD-L1-CHO-K cell line

The DNA fragment of full-length human PD-L1 (UniProtKB-Q9NZQ7, SEQ ID NO: 12) was synthesized by gene synthesis technology, and cloned into an expression vector. The vector was introduced into *Escherichia coli* by chemical transformation. The monoclonal *Escherichia coli* colonies were picked out, and sequencing was carried out to obtain a plasmid clone with a correct sequence. Plasmids were extracted and sequencing was carried out again for confirmation. CHO-K (Thermo, A1461801) cells were cultured with CD-CHO serum-free medium (Gibco, 10743029). One day before electroporation, the cells were passaged to reach 5×10⁶ cells/mL. The next day, the constructed plasmids were introduced into the CHO-K cells using Invitrogen's electroporation kit (Cat. no.: MPK10096) and electroporator (Cat. no.: MP922947). The electroporated cells were transferred to CD-CHO medium and incubated in a cell culture incubator at 37°C for 48 h. The electroporated CHO-K cells were plated into a 96-well plate at 2,000 cells/well, added with MSX (Millipore, GSS-1015-F) at a final concentration of 30 µM and GS supplement (Sigma, 58672C-100ml), placed and cultured in a carbon dioxide incubator at 37°C. 10 days later, a medium containing 30 µM MSX and 1×GS supplement was supplemented. The single cell clones grown in the 96-well plate were transferred to a 24-well plate for further expansion culture. Thereafter, after identification with FACS, a stable transfected cell line huPD-L1-CHO-K was successfully obtained.

### 1.3.4 Construction of CD3L-PD-L1-CHO cell line

The CD3L-PD-L1-CHO cell line was constructed based on the CHO cell line (CD3L-CHO cell) stably expressing CD3L (membrane-anchored anti-CD3-scFv, which is capable of directly stimulating CD3 signaling) which had been prepared by the applicant. Specifically, the plasmid expressing full-length human PD-L1 was introduced into the CD3L-CHO cell by electroporation. An antibiotic was used to screen a monoclonal cell line. Finally, a cell line stably expressing human PD-L1 was identified by FACS and named CD3L-PD-L1-CHO cell line.

### 1.3.5 Construction of Jurkat-PD-1-NFAT cell line

The Jurkat-PD-1-NFAT cell line was constructed based on the Jurkat cell line (Jurkat-NFAT cell) stably transfected with an NFAT luciferase reporter gene plasmid (containing a luciferase reporter gene whose expression was driven by an NFAT response element) which had been prepared by the applicant. Specifically, the plasmid expressing full-length human PD-1 (UNIPROT: Q15116, SEQ ID NO: 13) was introduced into the Jurkat-NFAT cell by electroporation. Puromycin at a final concentration of 2 µg/mL was used to screen monoclonal cell lines. Then, a cell line stably expressing human PD-1 was identified by FACS and named Jurkat-PD-1-NFAT cell line.

### Example 2 Configuration design and construction of bispecific antibodies against PD-L1 and IL-1

This Example described the structures of the exemplary bispecific antibodies against PD-L1 and blocking IL-1 activity, and construction of expression vectors thereof. 24 structures were designed for the construct: an amino acid sequence of the antagonist protein which was antagonistic against IL-1R1 was from the antagonist protein Anakinra (SEQ ID NO: 14). The amino acid sequence of the anti-IL-1 antibody was from the anti-IL-1β antibody Canakinumab (with VH and VL being SEQ ID NO: 15 and SEQ ID NO: 16, respectively). An amino acid sequence of the anti-PD-L1 antibody was from Atezolizumab (with VH and VL being SEQ ID NO: 17 and SEQ ID NO: 18, respectively). In certain constructs, the amino acid sequence of the linker was (G₄S)₃G (SEQ ID NO: 19), (G₄S)₄G (SEQ ID NO: 20), (G₄S)₃ (SEQ ID NO: 21), (G₄S)₄ (SEQ ID NO: 22), TVAAPSVFIFPP (SEQ ID NO: 23), or ASTKGPSVFPLAP (SEQ ID NO: 24). The configurations of the antibodies were as shown in Figures 1A-1I and 2A-2J, and corresponding amino acid sequences were as shown in Table 1. Specific configurations of the antibodies were described below:
Construct BsAb1: containing two identical first polypeptide chains which comprised from N-terminus to C-terminus a VH domain of the anti-PD-L1 antibody Atezolizumab, and a heavy chain constant region domain of IgG1 (including an IgG1 heavy chain CH1, an IgG1 heavy chain hinge region, and an IgG1 heavy chain Fc); and containing two identical second polypeptide chains which comprised from N-terminus to C-terminus the IL-1R1 antagonist protein Anakinra, a linker (G₄S)₄G, a VL domain of Atezolizumab, and a CL domain of antibody κ light chain. BsAb1 had the format in Figure 1A.

Construct BsAb2: containing two identical first polypeptide chains which comprised from N-terminus to C-terminus Anakinra, a linker (G₄S)₄G, a VH domain of Atezolizumab, and a heavy chain constant region domain of IgG1 (including an IgG1 heavy chain CH1, an IgG1 heavy chain hinge region, and an IgG1 heavy chain Fc); and containing two identical second polypeptide chains which comprised from N-terminus to C-terminus a VL domain of Atezolizumab, and a CL domain of antibody κ light chain. Construct BsAb3 was different from the construct BsAb2 in the linker in the first polypeptide chain, in which the linker of BsAb3 was (G₄S)₃G. BsAb2 and BsAb3 had the format in Figure 1B.

Construct BsAb4: containing two identical first polypeptide chains which comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc); and containing two identical second polypeptide chains which comprised from N-terminus to C-terminus a VL domain of Atezolizumab, a CL domain of antibody κ light chain, a linker (G₄S)₄G, and Anakinra. BsAb4 had the format in Figure 1C.

Construct BsAb5: containing two identical first polypeptide chains which comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc), a linker (G₄S)₄G, and Anakinra; and containing two identical second polypeptide chains which comprised from N-terminus to C-terminus a VL domain of Atezolizumab and a CL domain of antibody κ light chain. Construct BsAb6 was different from the construct BsAb5 in the linker in the first polypeptide chain, in which the linker of BsAb6 was (G₄S)₃G. BsAb5 and BsAb6 had the format in Figure 1D.

Construct BsAb7: containing 3 types of polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus Anakinra, a linker (G₄S)₄G, a VH domain of Atezolizumab, and a heavy chain constant region domain of IgG1 (including an IgG1 heavy chain CH1, an IgG1 heavy chain hinge region, and an IgG1 heavy chain Fc with hole mutation). Two identical third polypeptide chains comprised from N-terminus to C-terminus a VL domain of Atezolizumab and a CL domain of antibody κ light chain. BsAb7 had the format in Figure 1E.

Construct BsAb8: comprising 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VL domain of Atezolizumab and a CL domain of antibody κ light chain. A third polypeptide chain comprised from N-terminus to C-terminus a VH domain of Atezolizumab, a CL domain of antibody κ light chain, and an IgG1 heavy chain Fc with hole mutation. A fourth polypeptide chain comprised from N-terminus to C-terminus Anakinra, a linker (G₄S)₄G, a VL domain of Atezolizumab, an antibody IgG1 heavy chain CH1, and an IgG1 heavy chain hinge region (EPKSC). BsAb8 had the format in Figure 1F.

Construct BsAb9: comprising 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VL domain of Atezolizumab and a CL domain of antibody κ light chain. A third polypeptide chain comprised from N-terminus to C-terminus a VL domain of Atezolizumab and a heavy chain constant region domain of IgG1 (including an IgG1 heavy chain CH1, an IgG1 heavy chain hinge region, and an IgG1 heavy chain Fc with hole mutation). A fourth polypeptide chain comprised from N-terminus to C-terminus a VH domain of Atezolizumab, a CL domain of antibody κ light chain, a linker (G₄S)₄G, and Anakinra. BsAb9 had the format in Figure 1G.

Construct BsAb10: comprising 3 types of polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with hole mutation), a linker (G₄S)₄G, and Anakinra. Two identical third polypeptide chains comprised from N-terminus to C-terminus a VL domain of Atezolizumab and a CL domain of antibody κ light chain. BsAb10 had the format in Figure 1H.

Construct BsAb11: comprising 3 types of polypeptide chains. A first polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with Knob mutation), a linker (G₄S)₃, a VH domain of Atezolizumab, an IgG1 heavy chain CH1, and an IgG1 heavy chain hinge region (EPKSC). A second polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with hole mutation), a linker (G₄S)₄G, and Anakinra. Three identical third polypeptide chains comprised from N-terminus to C-terminus a VL domain of Atezolizumab and a CL domain of antibody κ light chain. BsAb11 had the format in Figure 1I.

Construct BsAb12: containing two identical first polypeptide chains which comprised from N-terminus to C-terminus a VH domain of Canakinumab, a linker (G₄S)₃, a VH domain of Atezolizumab, and a heavy chain constant region domain of IgG1 (including an IgG1 heavy chain CH1, an IgG1 heavy chain hinge region, and an IgG1 heavy chain Fc); and containing two identical second polypeptide chains which comprised from N-terminus to C-terminus a VL domain of Canakinumab, a linker (G₄S)₃, a VL domain of Atezolizumab, and a CL domain of antibody κ light chain. Construct BsAb13 was different from the construct BsAb12 in the linkers in the first polypeptide chain and the second polypeptide chain, in which the linker in the first polypeptide chain of construct BsAb13 was ASTKGPSVFPLAP, and the linker in the second polypeptide chain of BsAb13 was TVAAPSVFIFPP. BsAb12 and BsAb13 had the format in Figure 2A.

Construct BsAb14: containing two identical first polypeptide chains which comprised from N-terminus to C-terminus a VH domain of Atezolizumab, a linker (G₄S)₃, a VH domain of Canakinumab, and a heavy chain constant region domain of IgG1 (including an IgG1 heavy chain CH1, an IgG1 heavy chain hinge region, and an IgG1 heavy chain Fc); and containing two identical second polypeptide chains which comprised from N-terminus to C-terminus a VL domain of Atezolizumab, a linker (G₄S)₃, a VL domain of Canakinumab, and a CL domain of antibody κ light chain. BsAb14 had the format in Figure 2A.

Construct BsAb15: containing two identical first polypeptide chains which comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc), a linker (G₄S)₃, and an Canakinumab scFv (VH-(G₄S)₃-VL); and containing two identical second polypeptide chains which comprised from N-terminus to C-terminus a VL domain of the antibody Atezolizumab and a CL domain of antibody κ light chain. BsAb15 had the format in Figure 2B.

Construct BsAb16: containing 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VL domain of Atezolizumab and a CL domain of antibody κ light chain. A third polypeptide chain comprised from N-terminus to C-terminus a VL domain of Canakinumab, a CL domain of antibody κ light chain, and an IgG1 heavy chain Fc with hole mutation. A fourth polypeptide chain comprised from N-terminus to C-terminus a VH domain of Canakinumab, an IgG1 heavy chain CH1, and an IgG1 heavy chain hinge region (EPKSC). BsAb16 had the format in Figure 2C.

Construct BsAb17: containing 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VL domain of Atezolizumab and a CL domain of antibody κ light chain. A third polypeptide chain comprised from N-terminus to C-terminus a VH domain of Canakinumab, a CL domain of antibody κ light chain, and an IgG1 heavy chain Fc with hole mutation. A fourth polypeptide chain comprised from N-terminus to C-terminus a VL domain of Canakinumab, an IgG1 heavy chain CH1, and an IgG1 heavy chain hinge region (EPKSC). BsAb17 had the format in Figure 2D.

Construct BsAb18: containing 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a VL domain of Atezolizumab and a CL domain of antibody κ light chain. A third polypeptide chain comprised from N-terminus to C-terminus a VL domain of Canakinumab and a heavy chain constant region domain of IgG1 (including an IgG1 heavy chain CH1, an IgG1 heavy chain hinge region, and an IgG1 heavy chain Fc with hole mutation). A fourth polypeptide chain comprised from N-terminus to C-terminus a VH domain of Canakinumab and a CL domain of antibody κ light chain. BsAb18 had the format in Figure 2E.

Construct BsAb19: containing 3 types of polypeptide chains. A first polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with Knob mutation), a linker (G₄S)₃, and a Canakinumab scFv (VH-(G₄S)₃-VL). A second polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with hole mutation). Two identical third polypeptide chains comprised from N-terminus to C-terminus a VL domain of Atezolizumab and a CL domain of antibody κ light chain. BsAb 19 had the format in Figure 2F.

Construct BsAb20: containing 3 types of polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with Knob mutation). A second polypeptide chain comprised from N-terminus to C-terminus a Canakinumab scFv (VH-(G₄S)₃-VL), a linker (G₄S)₃, an IgG1 heavy chain hinge region, and an IgG1 heavy chain Fc with hole mutation. A third polypeptide chain comprised from N-terminus to C-terminus a VL domain of Atezolizumab and a CL domain of antibody κ light chain. BsAb20 had the format in Figure 2G.

Construct BsAb21: containing 3 types of polypeptide chains. A first polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with Knob mutation), a VH domain of Atezolizumab, an IgG1 heavy chain CH1, and an IgG1 heavy chain hinge region (EPKSC). A second polypeptide chain comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc with hole mutation), a linker (G₄S)₃, and a Canakinumab scFv (VH-(G₄S)₃-VL). Three identical third polypeptide chains comprised from N-terminus to C-terminus a VL domain of Atezolizumab and a CL domain of antibody κ light chain. BsAb21 had the format in Figure 2H.

Construct BsAb22: containing 4 polypeptide chains. A first polypeptide chain comprised from N-terminus to C-terminus a VH domain of Atezolizumab and a heavy chain constant region domain of IgG1 (including an IgG1 heavy chain CH1, an IgG1 heavy chain hinge region, and an IgG1 heavy chain Fc with F405L mutation). A second polypeptide chain comprised from N-terminus to C-terminus a VL domain of Atezolizumab and a CL domain of antibody κ light chain. A third polypeptide chain comprised from N-terminus to C-terminus a VH domain of Canakinumab, a CL domain of antibody κ light chain, and an IgG1 heavy chain Fc with K409R mutation. A fourth polypeptide chain comprised from N-terminus to C-terminus a VL domain of Canakinumab, an IgG1 heavy chain CH1, and an IgG1 heavy chain hinge region (EPKSC). BsAb22 had the format in Figure 2I.

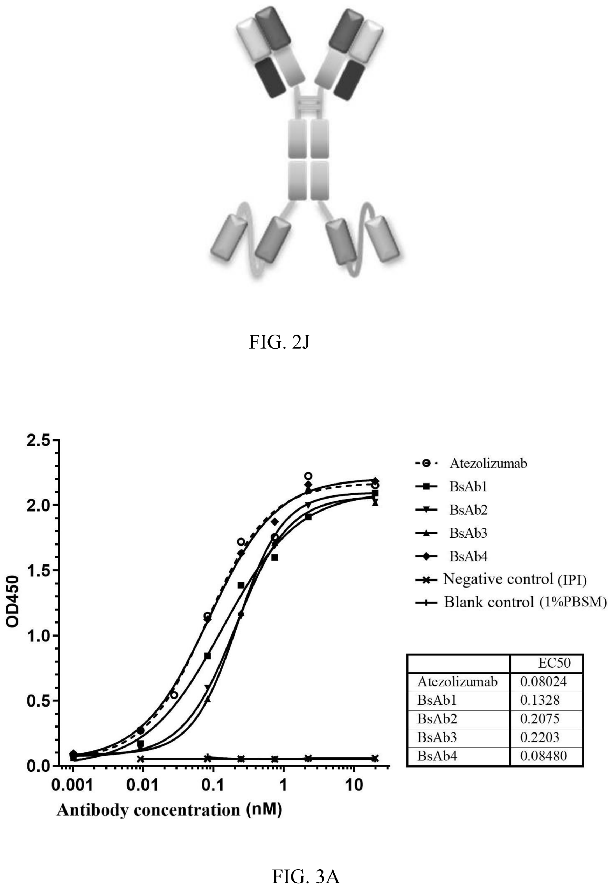

Construct BsAb23: containing two identical first polypeptide chains which comprised from N-terminus to C-terminus a VH domain of Atezolizumab, a heavy chain constant region domain of IgG1 (including an IgG1 heavy chain CH1, an IgG1 heavy chain hinge region, and an IgG1 heavy chain Fc), a linker (G₄S)₄, and an Canakinumab scFv (VH-(G4S)4-VL); and containing two identical second polypeptide chains which comprised from N-terminus to C-terminus a VL domain of Atezolizumab and a CL domain of antibody κ light chain. BsAb23 had the format in Figure 2J.

Construct BsAb25: containing two identical first polypeptide chains which comprised from N-terminus to C-terminus a VH domain of Canakinumab, a heavy chain constant region domain of IgG1 (including an IgG1 heavy chain CH1, an IgG1 heavy chain hinge region, and an IgG1 heavy chain Fc), a linker (G₄S)₄, and an Atezolizumab scFv (VH (with G44C mutation)-(G₄S)₄-VL (with Q100C mutation)); and containing two identical second polypeptide chains which comprised from N-terminus to C-terminus a VL domain of Canakinumab and a CL domain of antibody κ light chain. BsAb25 had the format in Figure 2J.

Construct BsAb26: containing two identical first polypeptide chains which comprised from N-terminus to C-terminus a VH domain of Atezolizumab and a heavy chain constant region domain of human IgG1 (including a human IgG1 heavy chain CH1, a human IgG1 heavy chain hinge region, and a human IgG1 heavy chain Fc (N297A)); and containing two identical second polypeptide chains which comprised from N-terminus to C-terminus a VL domain of Atezolizumab, a CL domain of antibody κ light chain, a linker (G₄S)₄G, and Anakinra. BsAb26 had the format in Figure 1C.

Construct BsAb27: containing two identical first polypeptide chains which comprised from N-terminus to C-terminus a VH domain of Atezolizumab, a heavy chain constant region domain of human IgG1 (including a human IgG1 heavy chain CH1, a human IgG1 heavy chain hinge region, and a human IgG1 heavy chain Fc (N297A)), a linker (G₄S)₃G, and Anakinra; and containing two identical second polypeptide chains which comprised from N-terminus to C-terminus a VL domain of Atezolizumab and a CL domain of antibody κ light chain. BsAb27 had the format in Figure 1D.

Construct BsAb28: containing two identical first polypeptide chains which comprised from N-terminus to C-terminus a VH domain of Atezolizumab, a linker (G₄S)₃, a VH domain of Canakinumab, and a heavy chain constant region domain of human IgG1 (including a human IgG1 heavy chain CH1, a human IgG1 heavy chain hinge region, and a human IgG1 heavy chain Fc (N297A)); and containing two identical second polypeptide chains which comprised from N-terminus to C-terminus a VL domain of Atezolizumab, a linker (G₄S)₃, a VL domain of Canakinumab, and a CL domain of antibody κ light chain. BsAb28 had the format in Figure 2A.

Construct BsAb29: containing two identical first polypeptide chains which comprised from N-terminus to C-terminus a VH domain of Canakinumab, a heavy chain constant region domain of human IgG1 (including a human IgG1 heavy chain CH1, a human IgG1 heavy chain hinge region, and a human IgG1 heavy chain Fc (N297A)), a linker (G₄S)₄, and an Atezolizumab scFv (VH (with G44C mutation)-(G₄S)₄-VL (with Q100C mutation)); and containing two identical second polypeptide chains which comprised from N-terminus to C-terminus a VL domain of Canakinumab and a CL domain of antibody κ light chain. BsAb29 had the format in Figure 2J.

Based on the structures of the constructs, PCR amplification was carried out to obtain the respective fragments, and the various fragments were connected through overlap extension PCR and then respectively incorporated into engineered eukaryotic expression vector plasmids pcDNA3.3-TOPO (Invitrogen) by homologous recombination, so as to constitute the complete full-length genes of polypeptide chains of the constructs. Constructed vectors containing the full-length genes of polypeptide chains of the constructs were separately transformed into *Escherichia coli* SS320 and cultured overnight at 37°C. Plasmids were extracted using an endotoxin-free plasmid extraction kit (OMEGA, D6950-01) to obtain endotoxin-free plasmids of the construct polypeptide chainsfor use in eukaryotic expression.

**Table 1 The amino acid sequences of polypeptide chains of the bispecific antibodies against PD-L1 and IL-1 (SEQ ID NO)**

| Name of antibody | First polypeptide chain | Second polypeptide chain | Third polypeptide chain | Fourth polypeptide chain |
|---|---|---|---|---|
| BsAb1 | 25 | 26 | NA | NA |
| BsAb2 | 27 | 28 | NA | NA |
| BsAb3 | 29 | 28 | NA | NA |
| BsAb4 | 25 | 30 | NA | NA |
| BsAb5 | 32 | 28 | NA | NA |
| BsAb6 | 32 | 28 | NA | NA |
| BsAb7 | 33 | 34 | 28 | NA |
| BsAb8 | 33 | 28 | 35 | 36 |
| BsAb9 | 33 | 28 | 37 | 38 |
| BsAb10 | 33 | 39 | 28 | NA |
| BsAb11 | 40 | 39 | 28 | NA |
| BsAb12 | 41 | 42 | NA | NA |
| BsAb13 | 43 | 44 | NA | NA |
| BsAb14 | 45 | 46 | NA | NA |
| BsAb15 | 47 | 28 | NA | NA |
| BsAb16 | 33 | 28 | 48 | 49 |
| BsAb17 | 33 | 28 | 50 | 51 |
| BsAb18 | 33 | 28 | 52 | 53 |
| BsAb19 | 54 | 55 | 28 | NA |
| BsAb20 | 33 | 56 | 28 | NA |
| BsAb21 | 40 | 57 | 28 | NA |
| BsAb22 | 58 | 28 | 59 | 60 |
| BsAb23 | 61 | 28 | NA | NA |
| BsAb25 | 62 | 63 | NA | NA |
| BsAb26 | 69 | 30 | NA | NA |
| BsAb27 | 70 | 28 | NA | NA |
| BsAb28 | 71 | 46 | NA | NA |
| BsAb29 | 72 | 63 | NA | NA |

### Example 3 Expression, purification, and physicochemical properties analysis of bispecific antibodies against PD-L1 and IL-1

### 3.1 Expression and purification of bispecific antibodies against PD-L1 and IL-1

The antibodies in Example 2 were expressed with the ExpiCHO Transient Transfection Expression System (Thermo Fisher, A29133). The specific method was as follows: on the day of transfection, it was confirmed that the density of CHO cells was about 7×10⁶-1×10⁷ cells/mL with the cell viability being > 98%. At this point, cells were adjusted to a final concentration of 6×10⁶ cell/mL with fresh ExpiCHO expression medium pre-warmed at 37°C. Plasmids of interest were diluted with OptiPROTM SFM pre-cooled at 4°C (1 µg of plasmids were added to 1 mL of the medium), while ExpiFectamineTM CHO was diluted with OptiPROTM SFM. Then, the two were mixed in equal volumes and gently pipetted to mix homogeneously, so as to prepare an ExpiFectamineTM CHO/plasmid DNA mixture. The mixture was incubated at room temperature for 1-5 min, slowly added to a prepared cell suspension with gently shaking, and finally placed in a cell culture shaker and cultured at 37°C and 8% CO₂. 18-22 h after transfection, ExpiCHOTM Enhancer and ExpiCHOTM Feed were added to the culture broth. Shaking flasks were placed in a shaker to continue culture at 32°C and 5% CO₂. On day 5 after transfection, the same volume of ExpiCHOTM Feed was slowly added while gently and evenly mixing the cell suspension. After 7-15 days post transfection, the cell culture supernatants with expressed proteins of interest were centrifuged at a high speed of 15,000 g for 10 min. The resulting supernatants were subjected to affinity purification with MabSelect SuRe LX (GE, 17547403). Then the proteins of interest were eluted with 100 mM sodium acetate (pH3.0), and then neutralized with 1 M Tris-HCl. Finally, the resulting proteins were exchanged into a PBS buffer through an ultrafiltration concentration tube (Millipore, UFC901096).

### 3.2 Determination of concentrations of bispecific antibodies against PD-L1 and IL-1

The concentrations of the purified bispecific antibodies in Example 3.1 were determined by an ultra-micro spectrophotometer (Hangzhou Allsheng Instrument Co., Ltd., Nano-300). The values obtained by dividing the A280 values as determined by theoretical extinction coefficients of the antibodies were used as the antibody concentration values for subsequent researches. After passing the quality inspection, the antibodies were aliquoted and stored at -80°C.

### 3.3 SDS-PAGE assay of bispecific antibodies against PD-L1 and IL-1

Preparation of non-reducing solutions: 1 µg of each of candidate antibodies and the reference IPI was taken, added with a 5×SDS loading buffer and 40 mM iodoacetamide, and heated in a dry bath at 75°C for 10 min, cooled to room temperature, and then centrifuged at 12,000 rpm for 5 min to collect supernatants.

Preparation of reducing solutions: 2 µg of respective candidate antibodies and the reference IPI were added with a 5×SDS loading buffer and 5 mM DTT, and heated in a dry bath at 100°C for 10 min, cooled to room temperature, and centrifuged at 12,000 rpm for 5 min to collect supernatants.

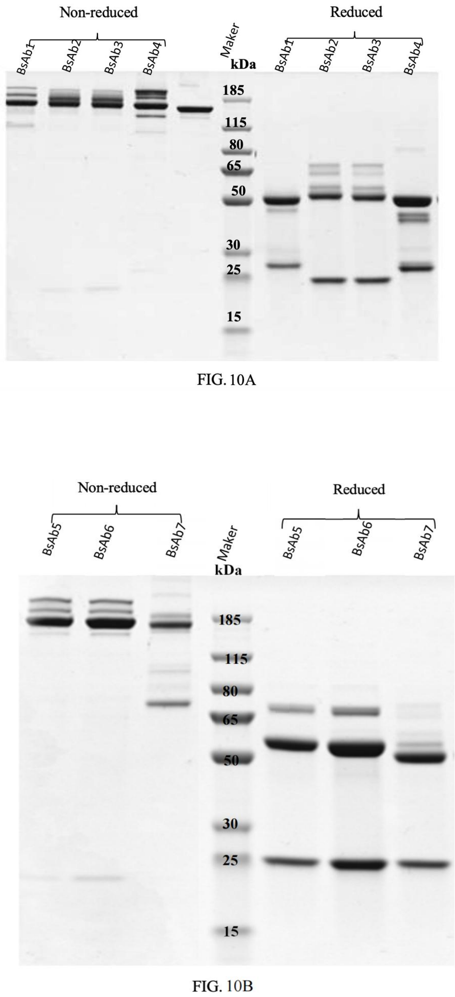

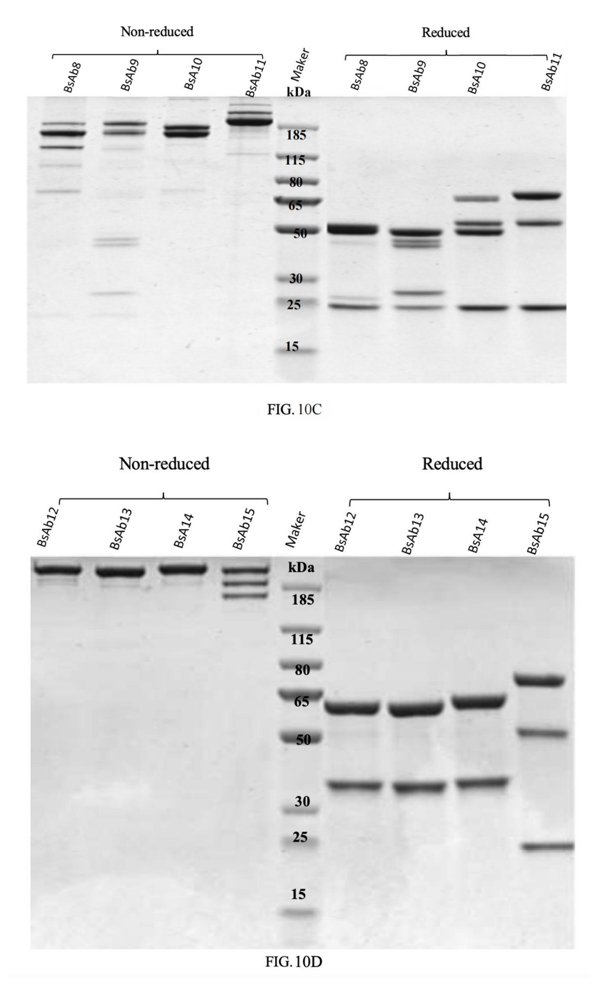

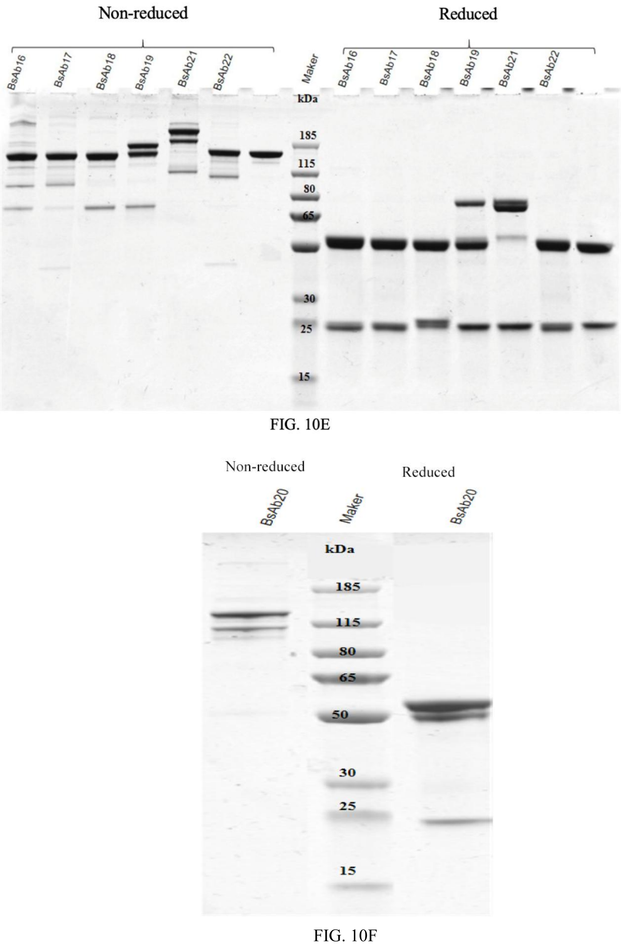

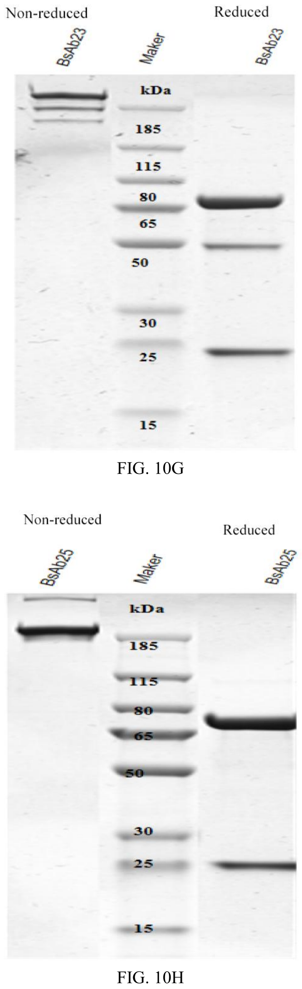

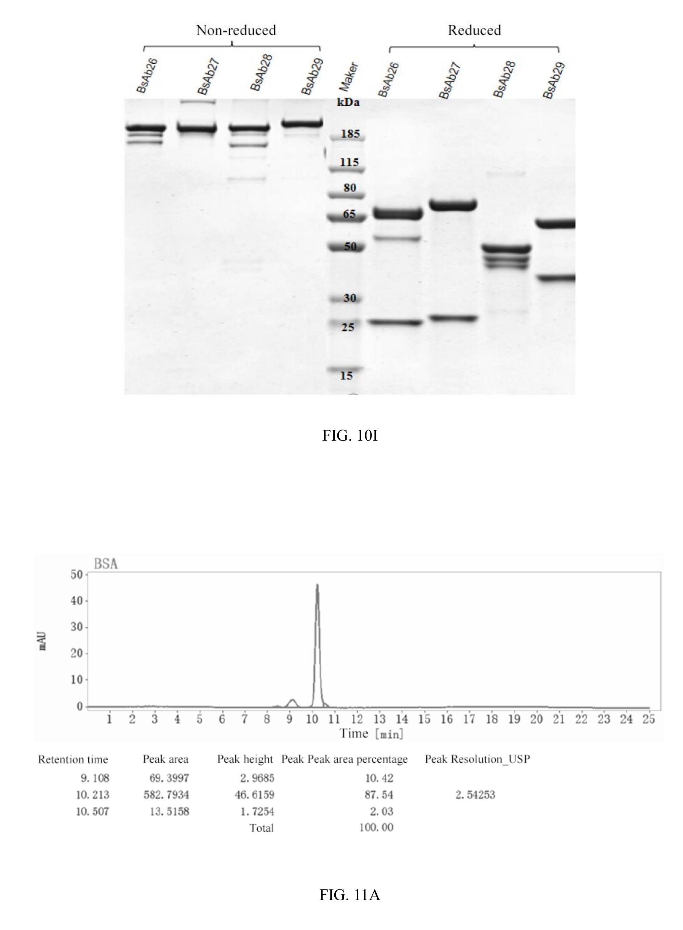

The supernatants were added to Bis-tris 4-15% gradient gels (purchased from GenScript) to perform gel electrophoresis at a constant voltage of 110 V, and the protein bands were visualized by Coomassie brilliant blue staining. After destaining, the protein gels were scanned with an EPSON V550 color scanner. The purities of reducing and non-reducing bands were calculated by ImageJ according to a peak area normalization method. Results were shown in Table 2 and Figures 10A-10I, and the bands of the candidate bispecific antibodies and the reference IPI in non-reducing gels all met the expected size, and the purities of BsAb12, BsAb13, BsAb14, BsAb16, BsAb22, BsAb25, BsAb28, and BsAb29 were greater than 95%.

### 3.4 SEC-HPLC assay of bispecific antibodies against PD-L1 and IL-1

Preparation of materials: 1. Mobile phase: 150 mmol/L phosphate buffer, pH 7.4. 2. Preparation of samples: each of the antibodies or the quality control IPI was diluted to 0.5 mg/mL with the mobile phase solution.

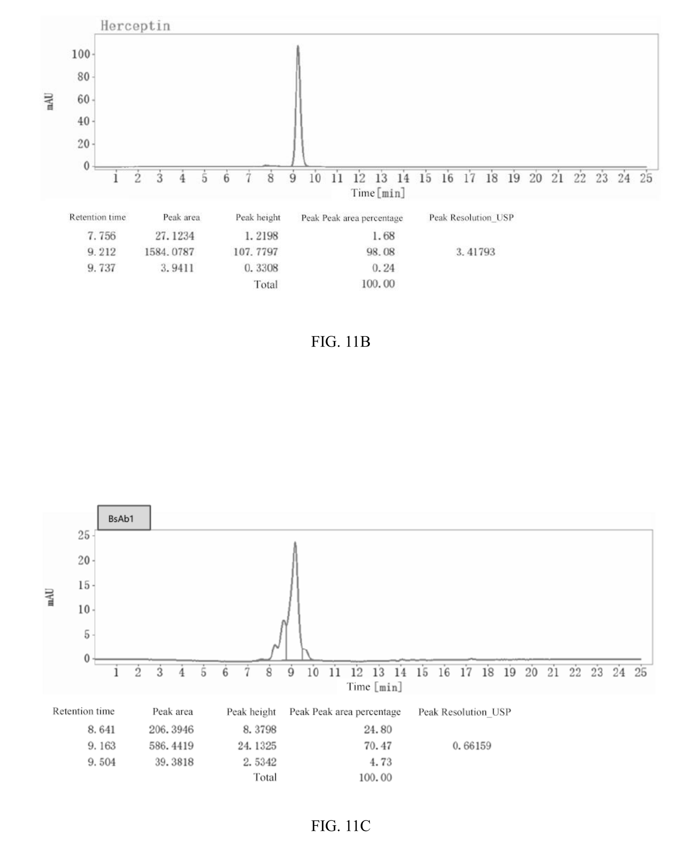

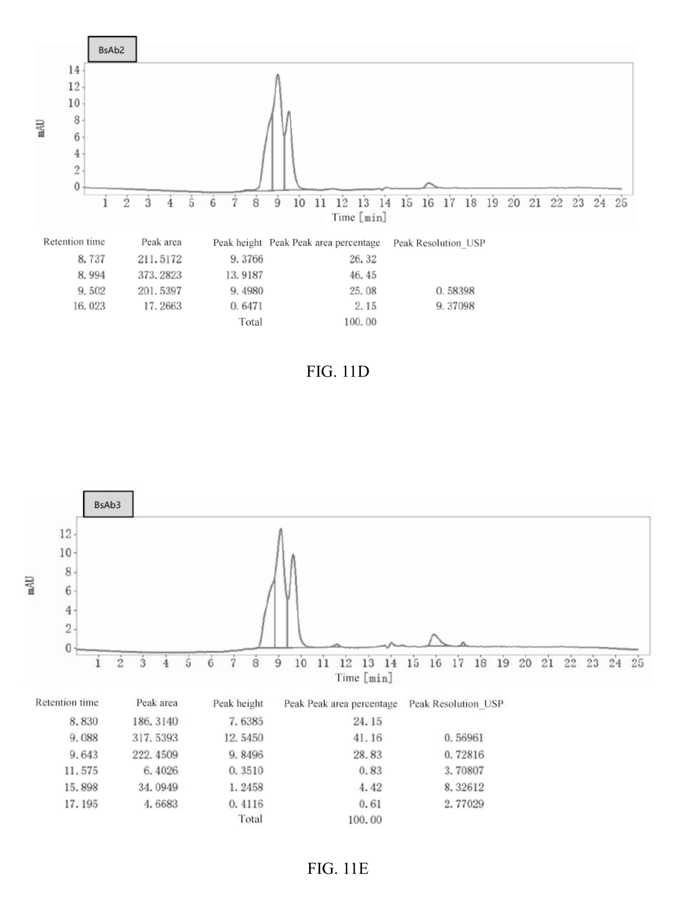

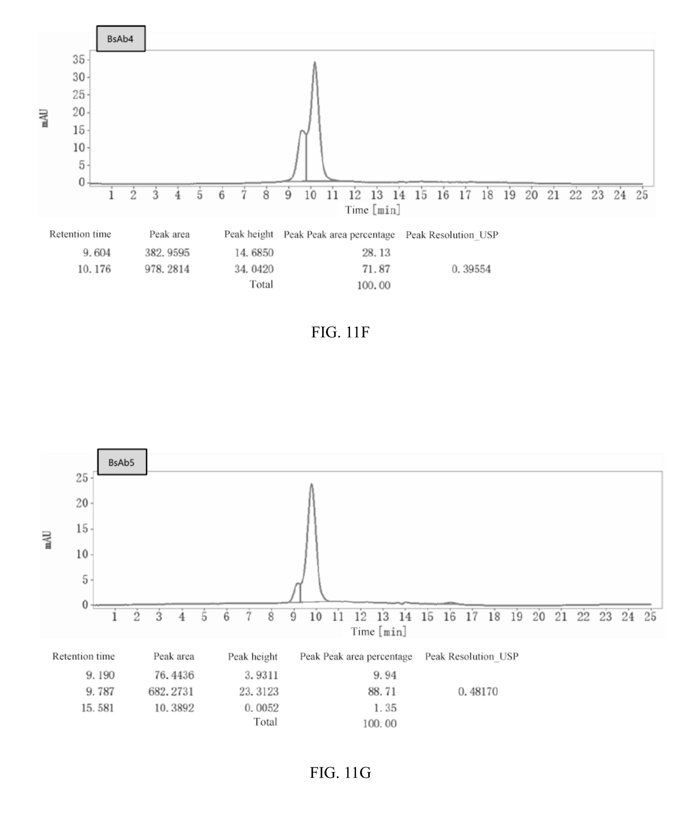

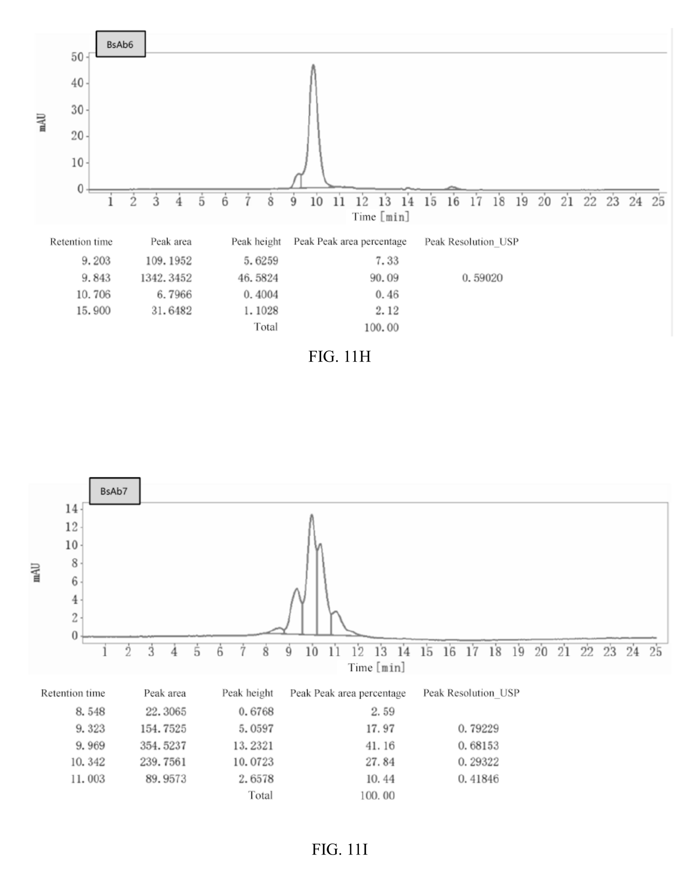

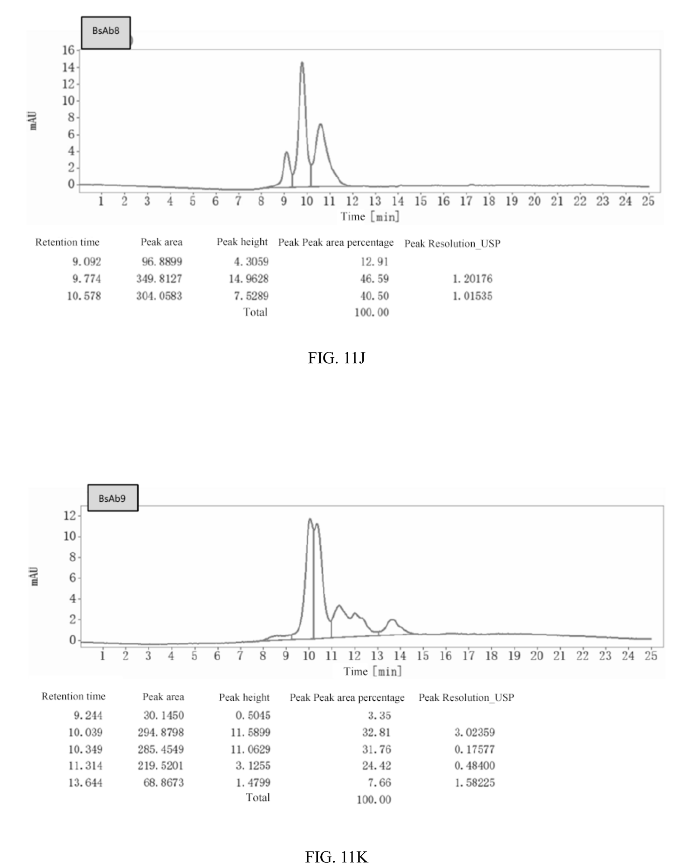

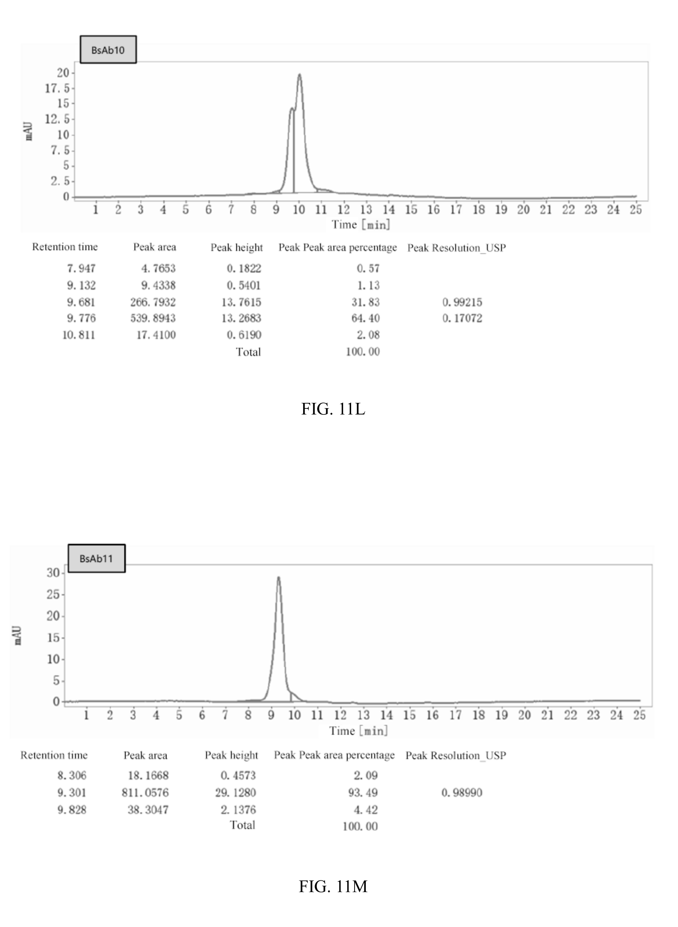

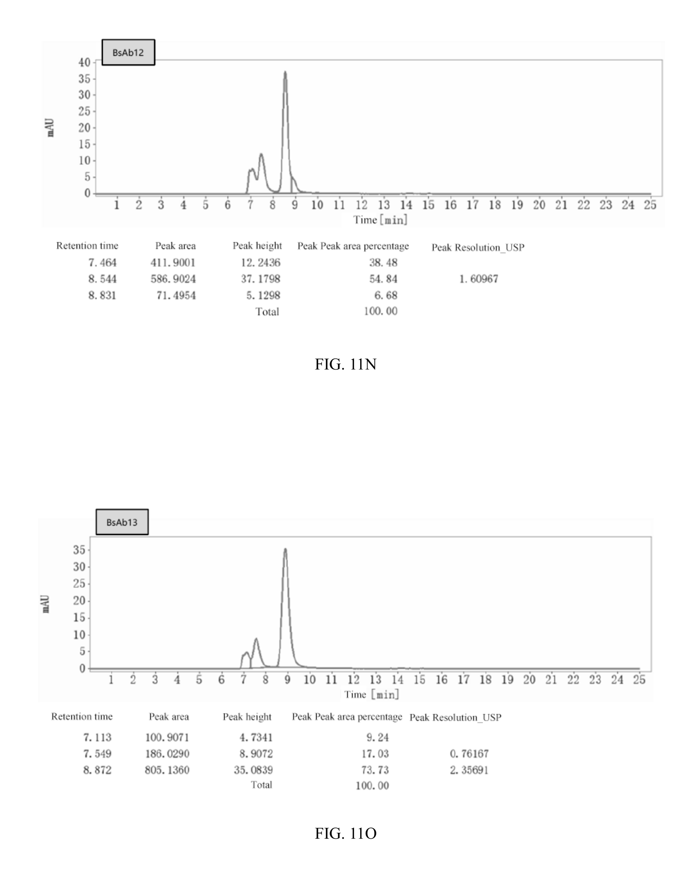

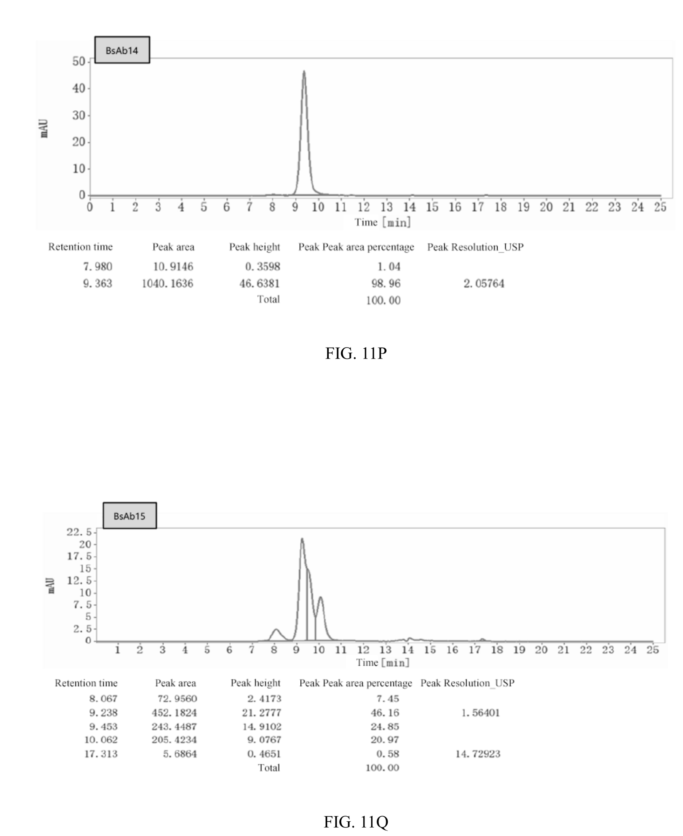

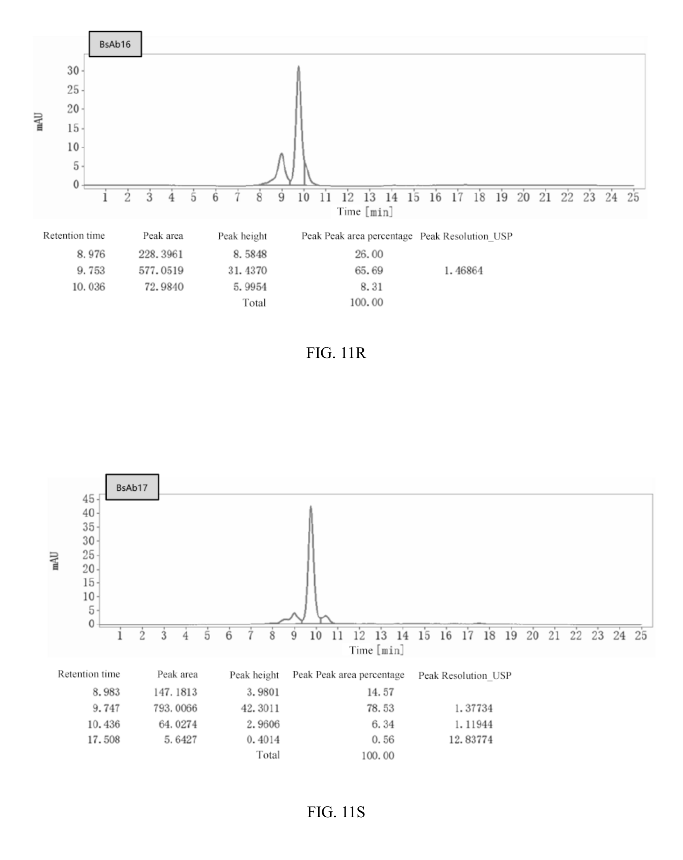

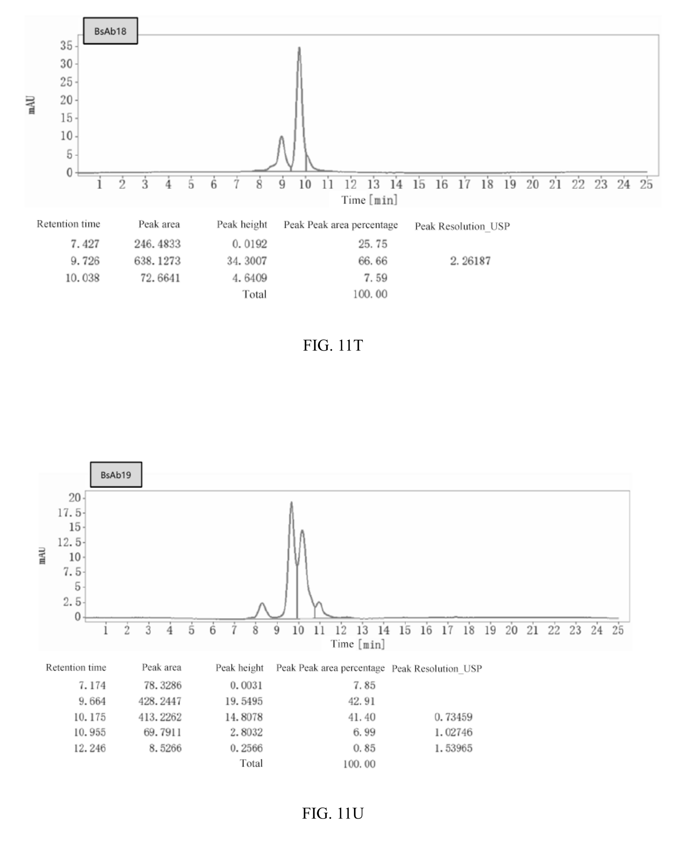

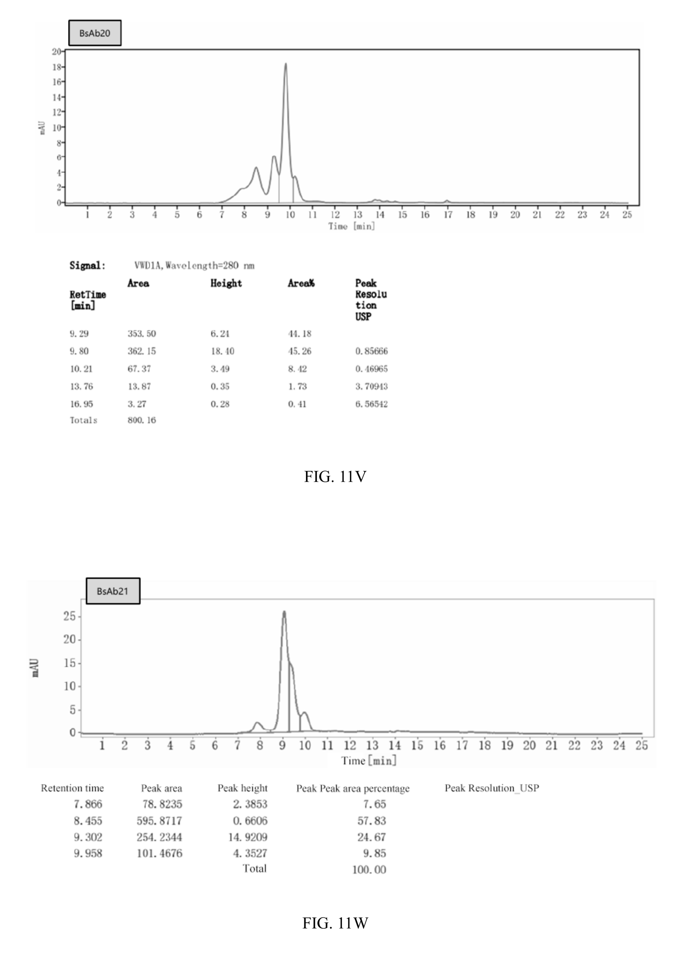

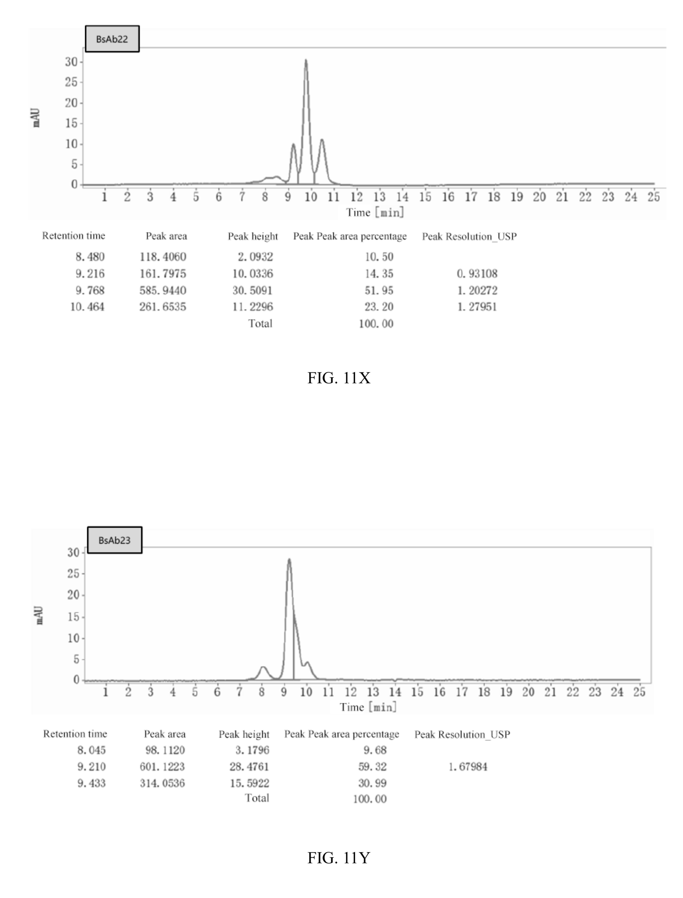

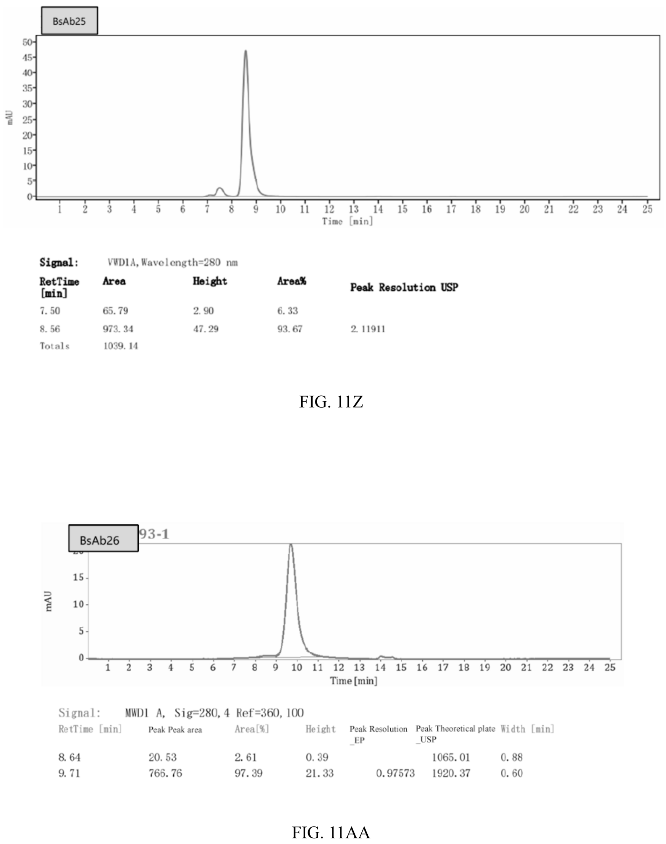

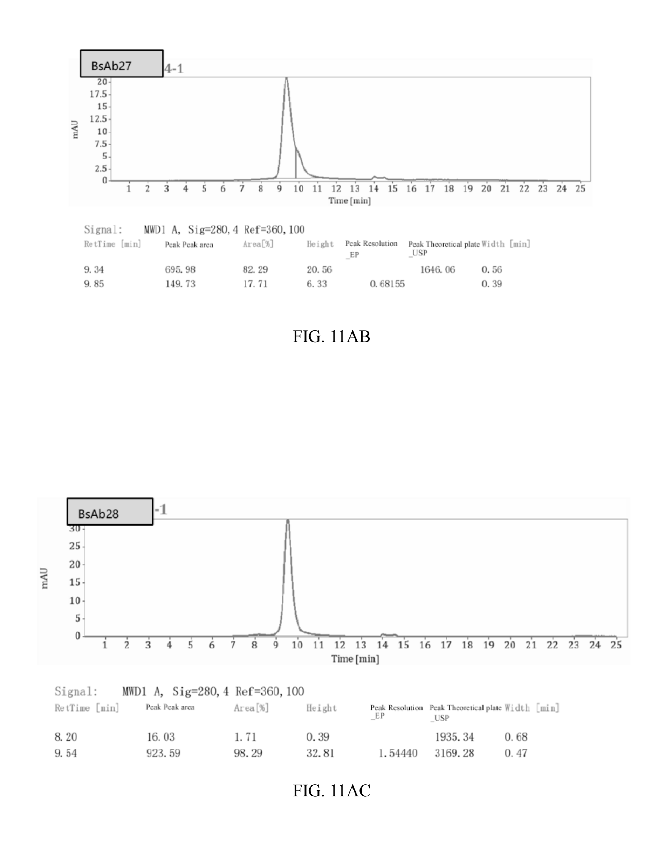

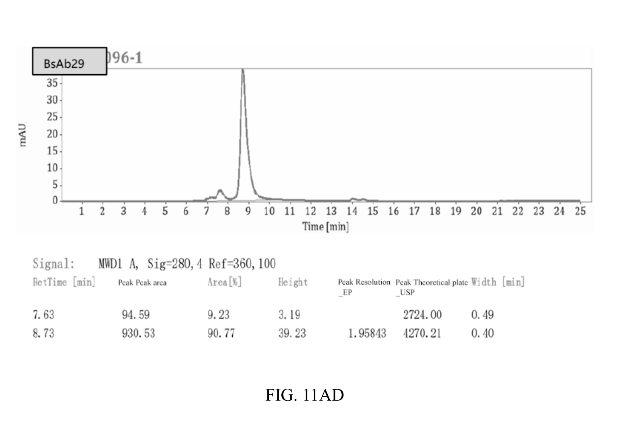

An Agilent HPLC 1100 or Shimadzu LC2030C PLUS liquid chromatograph was used with an XBridge BEH column (SEC 3.5 µm, 7.8 mm I.D.×30 cm), a Waters flow rate set at 0.8 mL/min, an injection volume of 20 µL, and wavelengths of 280 nm and 214 nm for VWD detector. A blank solution, an IPI quality control solution, and an antibody sample solution were injected sequentially. Percentages of high molecular weight polymers, antibody monomers, and low molecular weight substances in the samples were calculated using an area normalization method. Results showed that the purities of the monomers BsAb6, BsAb11, BsAb14, BsAb25, BsAb26, BsAb28, and BsAb29 were all greater than 90% (Table 2 and Figures 11A-11AD).

**Table 2 Preparation, and physiochemistry and purity data of bispecific antibodies against PD-L1 and IL-1**

| Name of antibody | Molecular weight (kDa) | Isoelectric point | Extinction coefficient | SDS-PAGE (%) | SEC-HPLC (%) |
|---|---|---|---|---|---|
| BsAb1 | 181.48 | 7.77 | 1.47 | 88.6 | 70.47 |
| BsAb2 | 181.46 | 7.77 | 1.47 | 75.5 | 46.45 |
| BsAb3 | 180.84 | 7.77 | 1.47 | 80.2 | 41.16 |
| BsAb4 | 181.48 | 7.77 | 1.47 | 70.9 | 71.87 |
| BsAb5 | 181.48 | 7.77 | 1.47 | 84 | 88.71 |
| BsAb6 | 180.86 | 7.77 | 1.47 | 82.4 | 90.09 |
| BsAb7 | 162.96 | 8.07 | 1.55 | 88.5 | Abnormal peak shape |
| BsAb8 | 163.04 | 7.99 | 1.55 | 91.2 | 46.59 |
| BsAb9 | 163.04 | 7.99 | 1.55 | Cannot be determined | Abnormal peak shape |
| BsAb10 | 162.96 | 8.07 | 1.55 | Cannot be determined | 64.4 |
| BsAb11 | 187.33 | 8.27 | 1.64 | Cannot be determined | 93.49 |
| BsAb12 | 197.76 | 8.31 | 1.65 | >95.0 | 54.84 |
| BsAb13 | 198.94 | 8.4 | 1.64 | >95.0 | 73.73 |
| BsAb14 | 197.9 | 8.31 | 1.65 | >95.0 | 98.96 |
| BsAb15 | 197.76 | 8.31 | 1.65 | Cannot be determined | 46.16 |
| BsAb16 | 144.75 | 8.32 | 1.54 | >95.0 | 65.69 |
| BsAb17 | 144.83 | 8.26 | 1.54 | 94.2 | 78.53 |
| BsAb18 | 144.83 | 8.26 | 1.54 | Cannot be determined | 66.66 |
| BsAb19 | 171.11 | 8.32 | 1.65 | Cannot be determined | 42.91 |
| BsAb20 | 124.34 | 8.18 | 1.62 | 72.4 | 45.26 |
| BsAb21 | 195.47 | 8.44 | 1.72 | 76.5 | 57.83 |
| BsAb22 | 145.02 | 8.28 | 1.52 | >95.0 | 51.95 |
| BsAb23 | 199.02 | 8.31 | 1.64 | 83.2 | 59.32 |
| BsAb25 | 199.2 | 8.28 | 1.64 | >95.0 | 93.67 |
| BsAb26 | 181.48 | 7.77 | 1.47 | Cannot be determined | 97.39 |
| BsAb27 | 180.86 | 7.77 | 1.47 | 87.2 | 82.29 |
| BsAb28 | 197.9 | 8.31 | 1.65 | >95.0 | 98.29 |
| BsAb29 | 199.2 | 8.28 | 1.64 | >95.0 | 90.77 |

### Example 4 Evaluation of antigen binding and blocking activities of bispecific antibodies against PD-L1 and IL-1 at protein level

### 5.1 Binding activities of candidate bispecific antibodies at protein level

This Example evaluated the antigen binding abilities of candidate bispecific antibody molecules against PD-L1 or IL-1. A specific experimental method was as follows:
96-well ELISA plates were coated with the human recombinant protein huPD-L1-His, huIL-1R1-His, or huIL-1β-His overnight at 4°C. The next day, the plates were washed with PBST for 3 times and then blocked with 5% skim milk for 2 h. The plates were washed with PBST for 3 times, and then added with different concentrations of test antibodies and incubated for 1 h. Thereafter, washing was carried out with PBST for 3 times, then the secondary antibody Anti-human-IgG-Fc-HRP (abcam, ab79225) was added and incubated for 1 h. After completion of incubation, the plates were washed with PBST for six times and added with TMB (SurModics, TMBS-1000-01) for color development. Based on the results of the color development, 2 M HCl was added to terminate reactions, and OD450 values were read by a microplate reader (Molecular Devices, SpecterMax 190). Data were analyzed using PRISM^{™} (GraphPad Software, San Diego, CA) and EC₅₀ values (nM) were calculated.

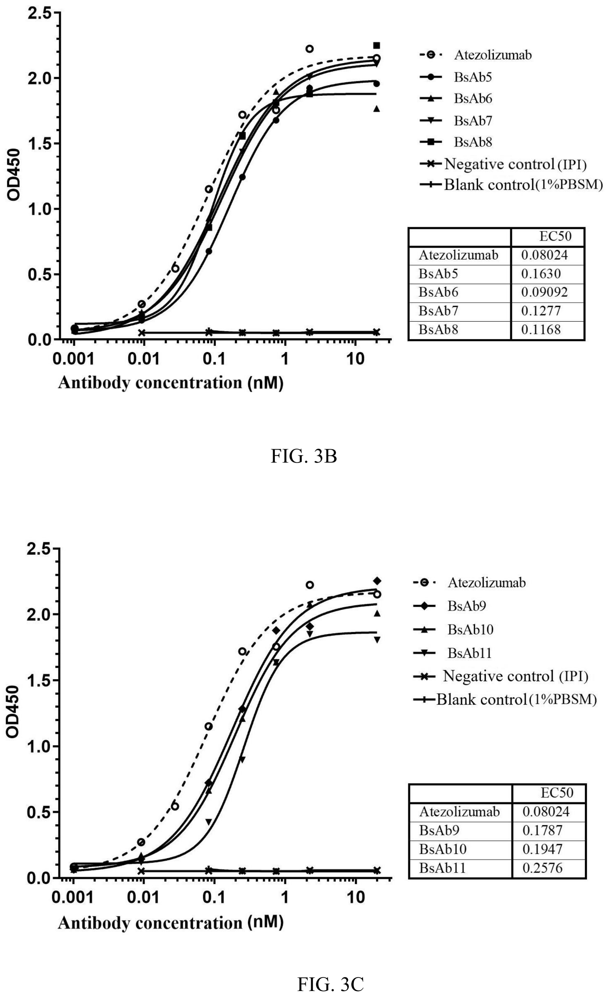

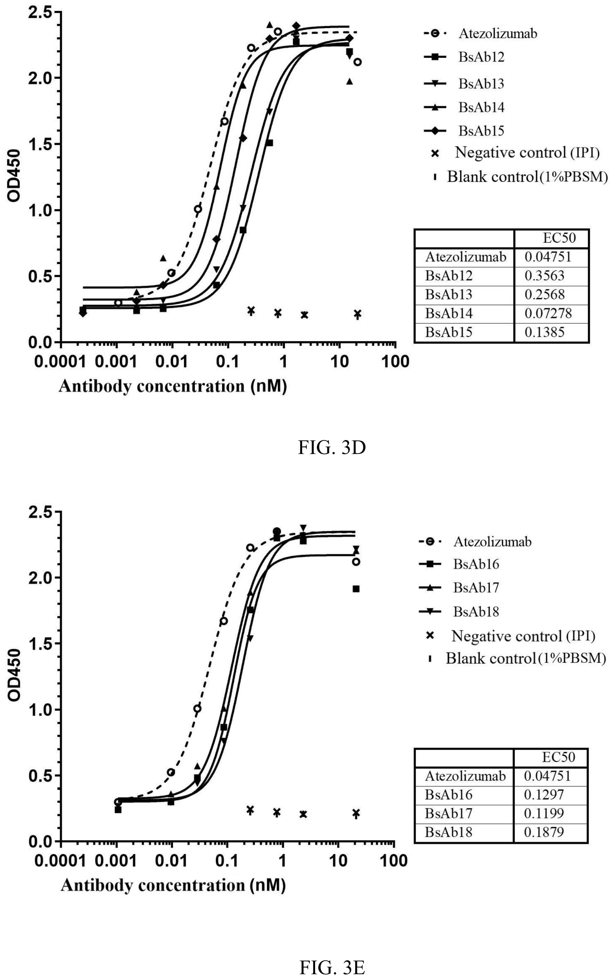

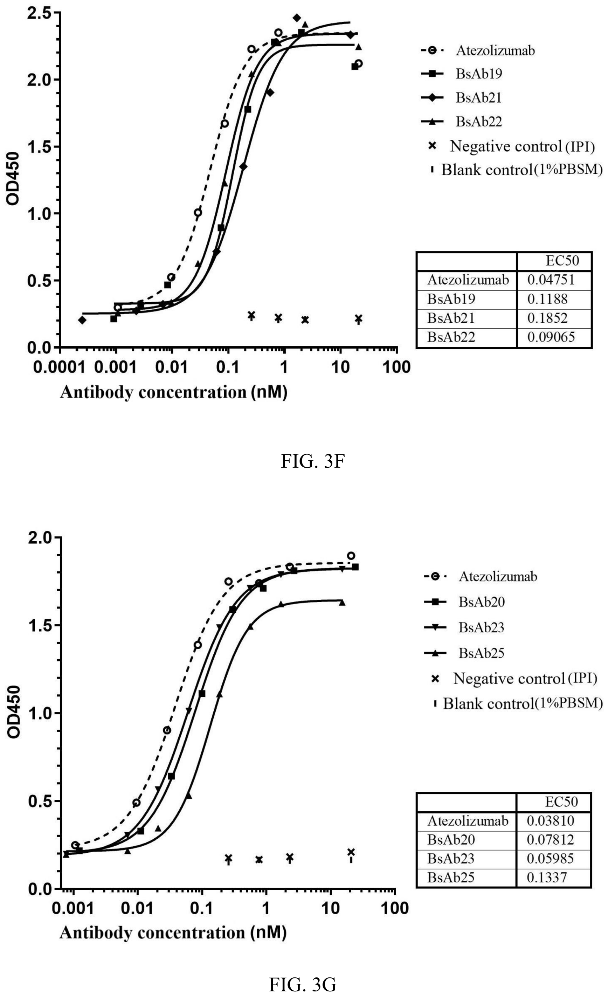

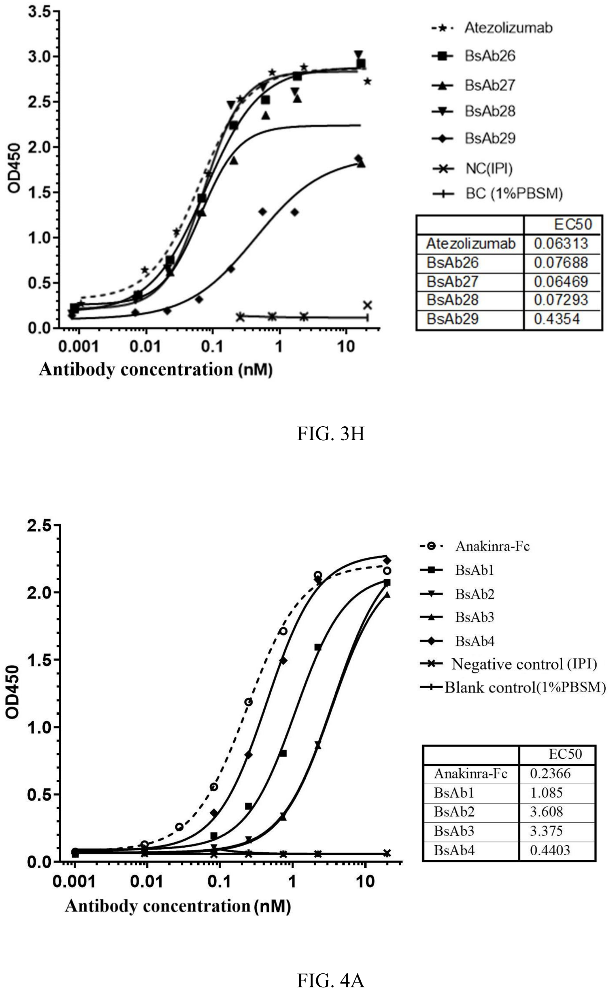

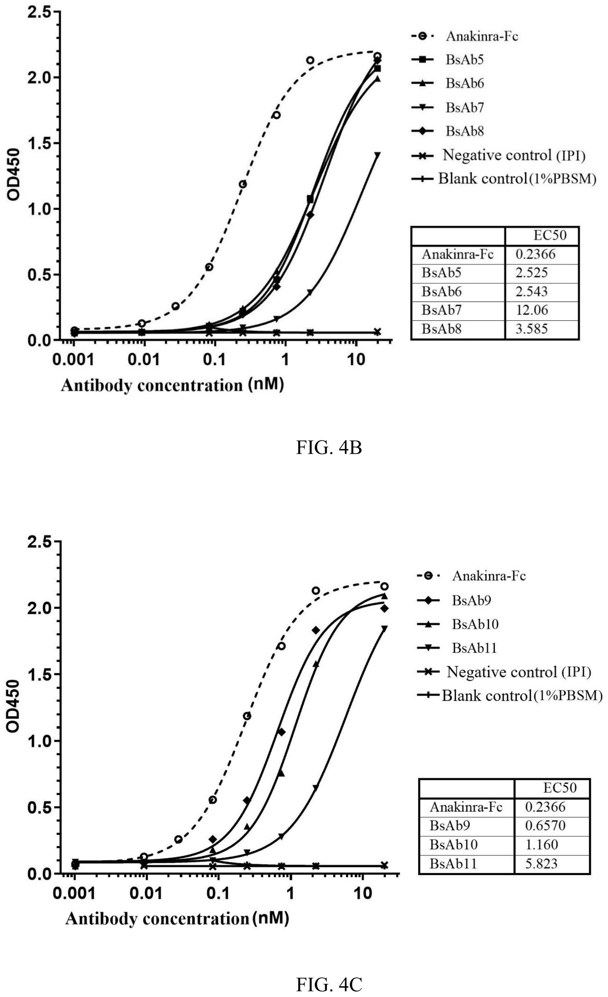

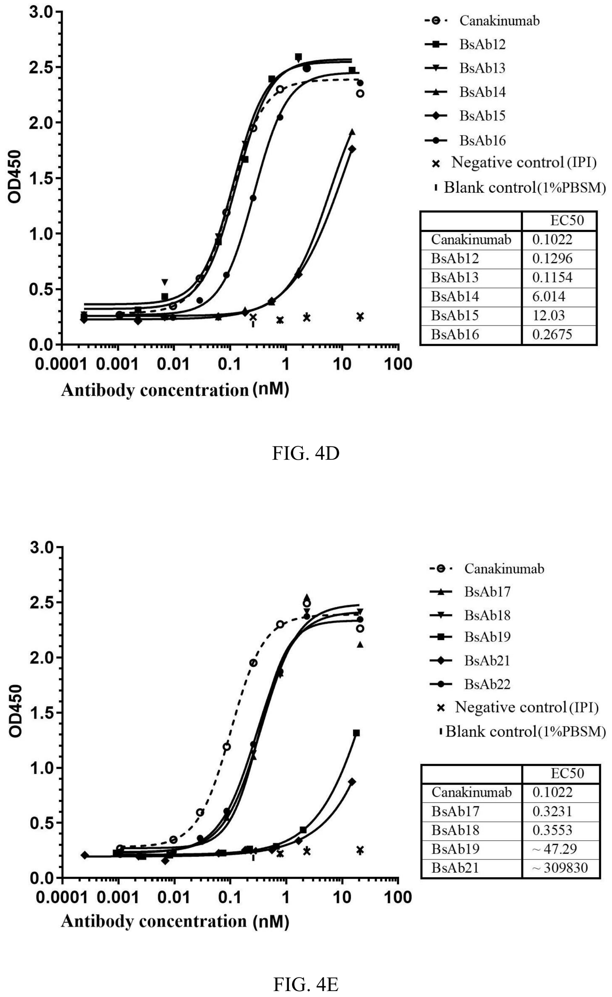

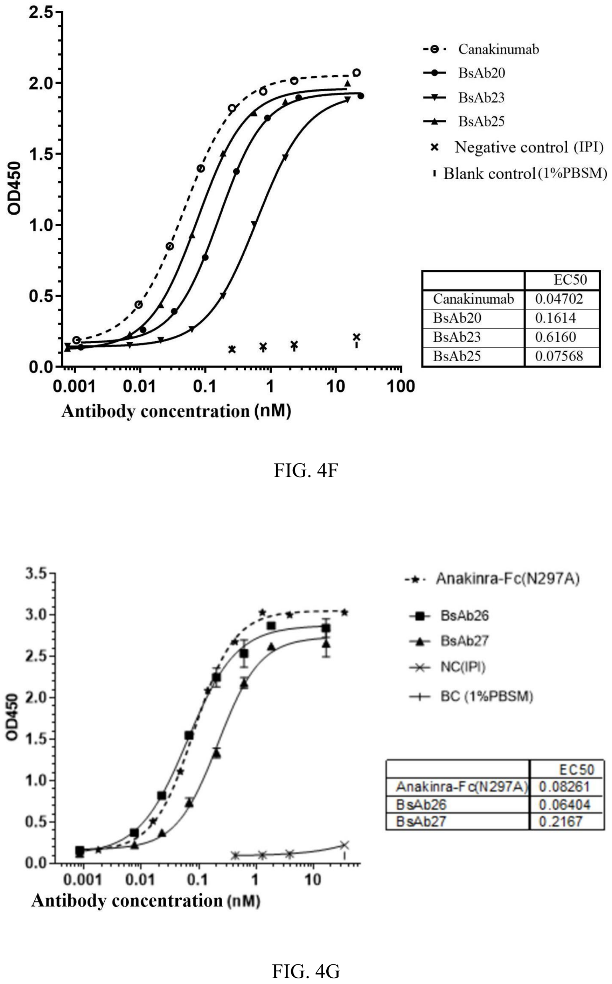

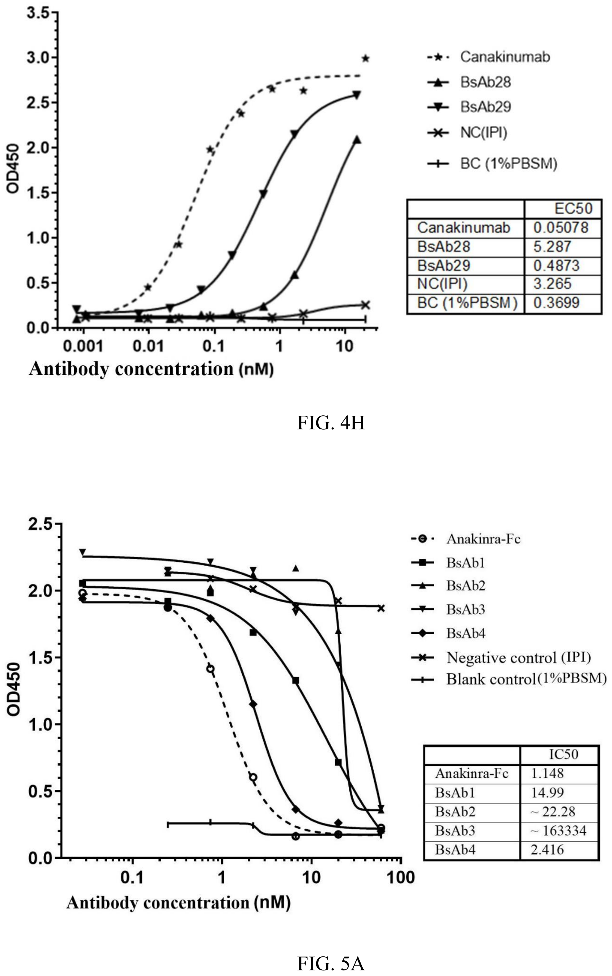

The binding abilities of the candidate bispecific antibodies to huPD-L1-His were shown in Figures 3A-3H. Each candidate molecule bound to the antigen PD-L1 with a high affinity, in which BsAb4, BsAb6, BsAb26, BsAb27, and BsAb28 showed the strongest binding, being comparable to the positive control antibody Atezolizumab, while the others showed weaker binding than the positive control antibody. In addition, as shown in Figures 4A-4H, the candidate bispecific antibody molecules all had certain binding abilities to huIL-1R1-His or IL-1β-His, in which BsAb12, BsAb13 and BsAb26 showed the strongest binding, being comparable to the positive control Canakinumab, while the others showed weaker binding than the positive control Anakinra-Fc or Canakinumab or Anakinra-Fc (N297A).

### 5.2 Activities of candidate bispecific antibody molecules in blocking IL-1β/IL-1R1 binding

The obtained bispecific antibodies were evaluated for their IL-1β/IL-1R1 blocking activities. ELISA method was used to determine whether the bispecific antibodies block the binding of huIL-1β to huIL-1R1. The specific method was as follows:
96-well ELISA plates were coated with the human recombinant protein huIL-1R1-Fc overnight at 4°C. The next day, the plates were washed with PBST for 3 times and then blocked with 5% skim milk at room temperature for 2 h. The plates were washed with PBST for 3 times, and then added with different concentrations of test antibodies and incubated at room temperature for 1 h. Washing was carried out with PBST for 3 times, and then the 96-well ELISA plates were added with huIL-1β-His and incubated at room temperature for 1 h. Thereafter, washing was carried out with PBST for 3 times, and then the secondary antibody Anti-6*His-HRP (proteintech, HRP-66005) was added and incubated at room temperature for 50 min. After completion of incubation, the plates were washed with PBST for 6 times and added with TMB (SurModics, TMBS-1000-01) for color development. Based on the results of the color development, 2 M HCl was added to terminate reactions, and OD450 values were read by a microplate reader (Molecular Devices, SpecterMax 190). Data were analyzed using PRISM^{™} (GraphPad Software, San Diego, CA) and IC₅₀ values (nM) were calculated.

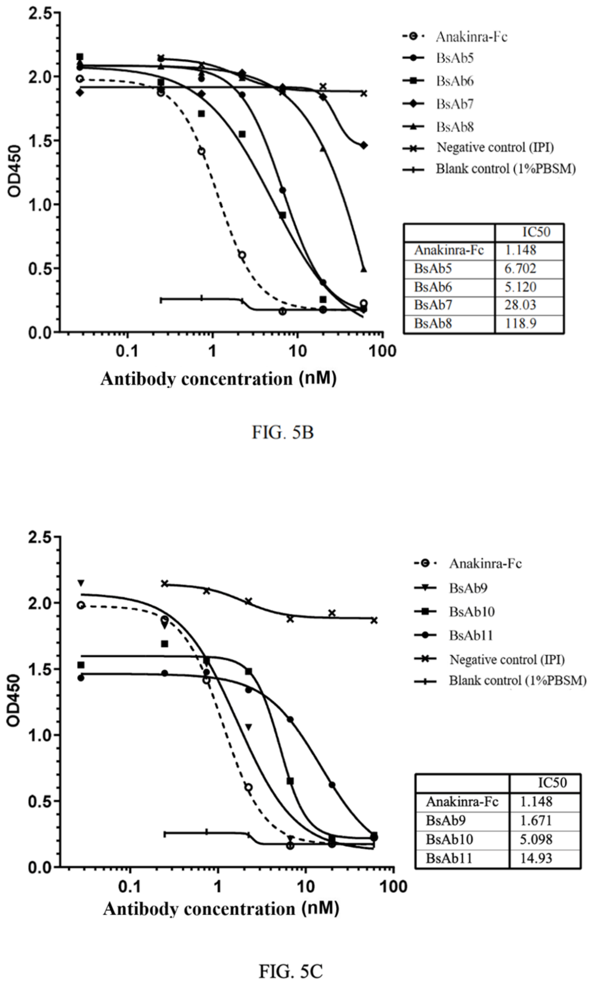

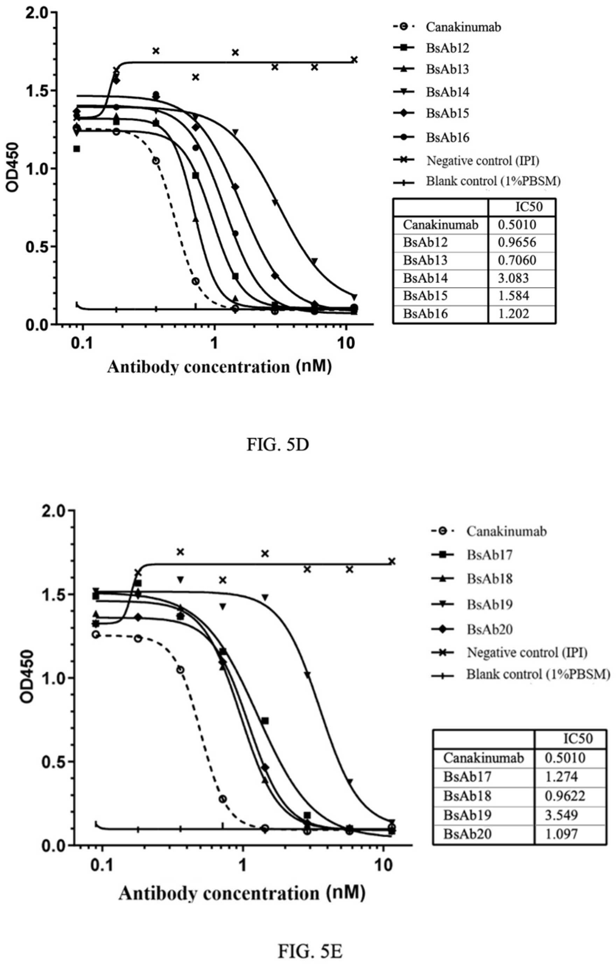

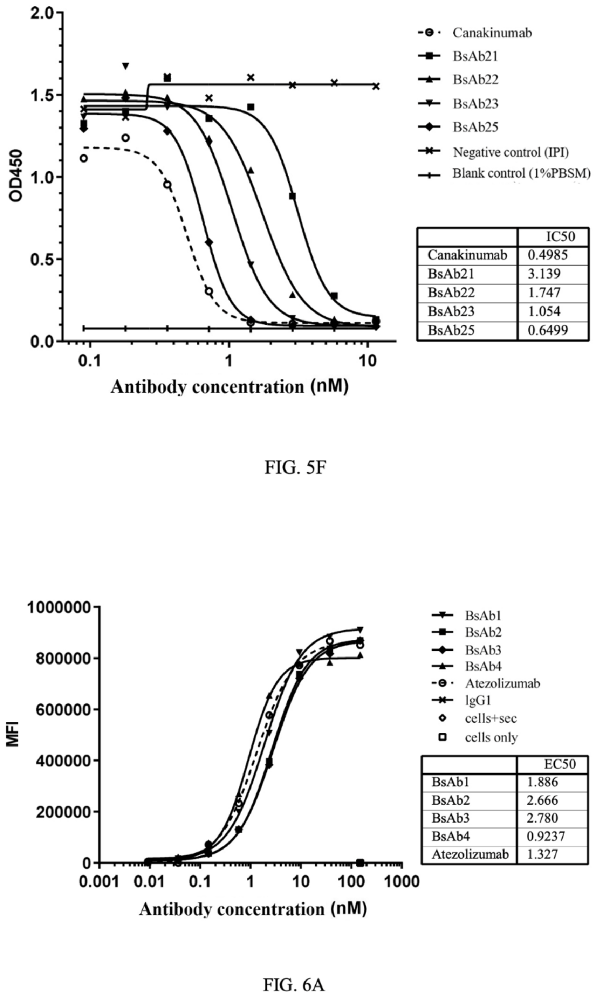

The activities of the candidate bispecific antibodies in blocking IL-1β/IL-1R1 were shown in Figures 5A-5F. The candidate molecules had activities in blocking IL-1β/IL-1R1 at protein level, which are however all weaker than the positive control Anakinra-Fc or Canakinumab.

### Example 6 Evaluation of binding and blocking activities of bispecific antibodies against PD-L1 and IL-1 at huPD-L1-CHO-K cell level

### 6.1 Evaluation of binding activities of candidate bispecific antibodies at huPD-L1-CHO-K cell level

Binding of the bispecific antibodies on huPD-L1-CHO-K cells was detected using FACS method (expressed as mean fluorescence intensity, MFI). The specific experimental method was as follows:
HuPD-L1-CHO-K cells with good culture condition were collected and centrifuged at 300 g to remove supernatants. The cells were resuspended with FACS buffer (PBS buffer added with 2% FBS) and counted, and densities of cell suspensions were adjusted. 1×10⁵ cells were added to each well and centrifuged again to remove supernatants. Then the test antibodies and the control antibody Atezolizumab serially diluted were added to corresponding wells. After incubation at 4°C for 60 min, the cells were rinsed with FACS buffer for three times. 0.5 µg of PE-labeled goat anti-human IgG Fc antibody (Abcam, ab98596) was added and incubated at 4°C for 30 min. Finally, the incubated cells were washed for three times, resuspended in 200 µL of FACS buffer, and subjected to detection with a flow cytometer (Beckman, CytoFLEX AOO-1-1102).

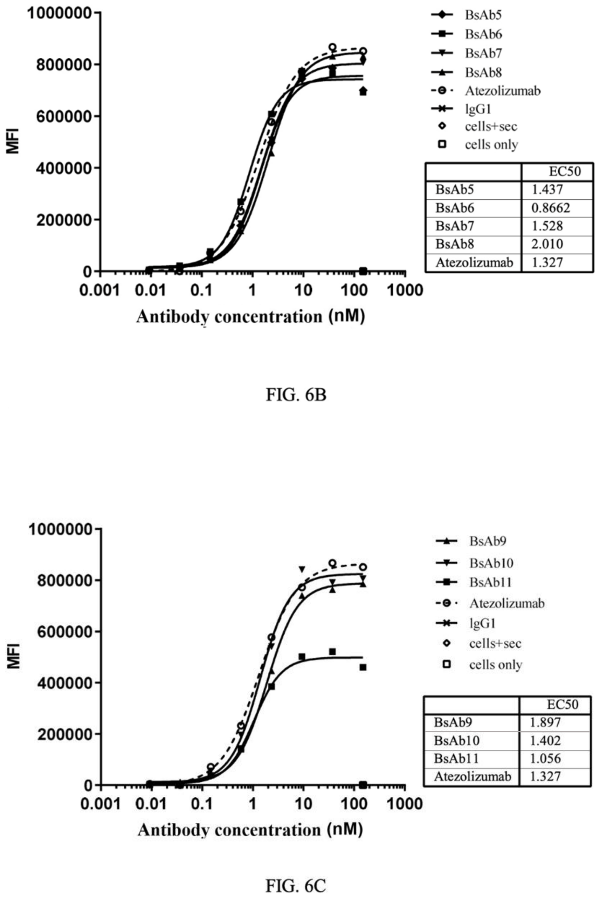

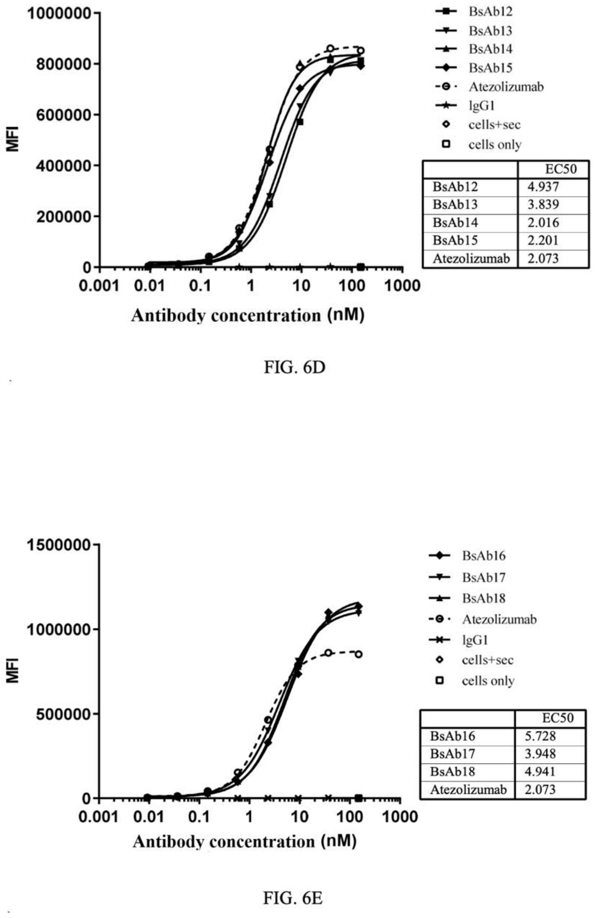

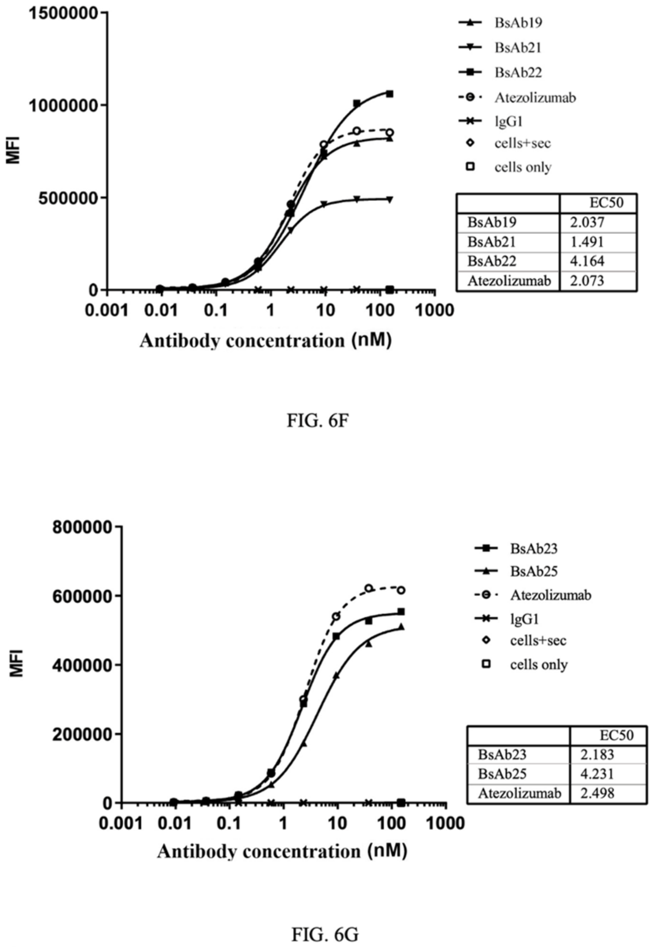

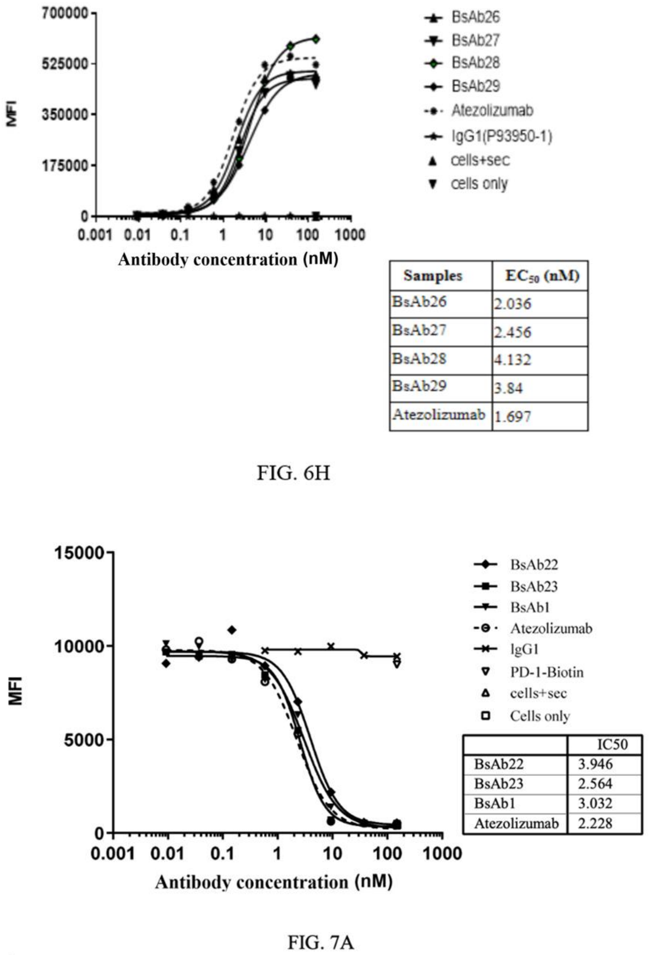

Results were shown in Figures 6A-6H. The bispecific antibodies bound to the huPD-L1-overexpressing-CHO-K cells with relatively high affinities, in which the binding activities of BsAb4 and BsAb6 were significantly stronger than those of other candidate antibodies and the positive control antibody Atezolizumab, while the binding activities of BsAb5, BsAb10, BsAb11, BsAb14, BsAb19, and BsAb23 were comparable to that of the positive control antibody Atezolizumab.

### 6.2 Assessment of activities of candidate bispecific antibodies in blocking PD-1/PD-L1 at cell level

Activities of the bispecific antibodies in blocking PD-1/PD-L1 binding at cell level were determined and assessed using FACS method (expressed as mean fluorescence intensity, MFI). The specific experimental method was as follows:
HuPD-L1-CHO-K cells with good culture condition were collected and centrifuged at 300 g to remove supernatants. The cells were resuspended with FACS buffer (PBS buffer added with 2% FBS) and counted, and densities of cell suspensions were adjusted. 1×10⁵ cells were added to each well and centrifuged again to remove supernatants. Then the test antibodies and the control antibody Atezolizumab serially dilute were added to corresponding wells and incubated at 4°C for 60 min. After the cells were washed twice with FACS buffer, a biotin-labeled PD-1-biotin protein was added and incubated at 4 °C for 60 min. Thereafter, the cells were washed twice with FACS buffer, added with PE-labeled streptavidin (Invitrogen, 12-4317-087) and incubated at 4°C for 30 min. The cells in incubated mixtures were washed for 3 times, then resuspended in 200 µL of FACS buffer, and finally subjected to detection with a flow cytometer (Beckman, CytoFLEX AOO-1-1102). Data were analyzed using PRISMTM (GraphPad Software, San Diego, CA) and IC50 values were calculated.

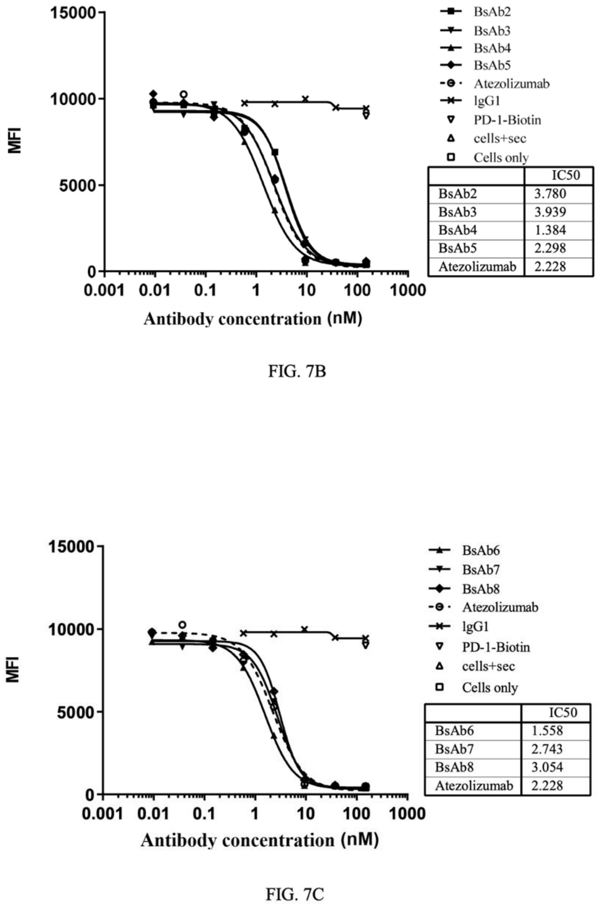

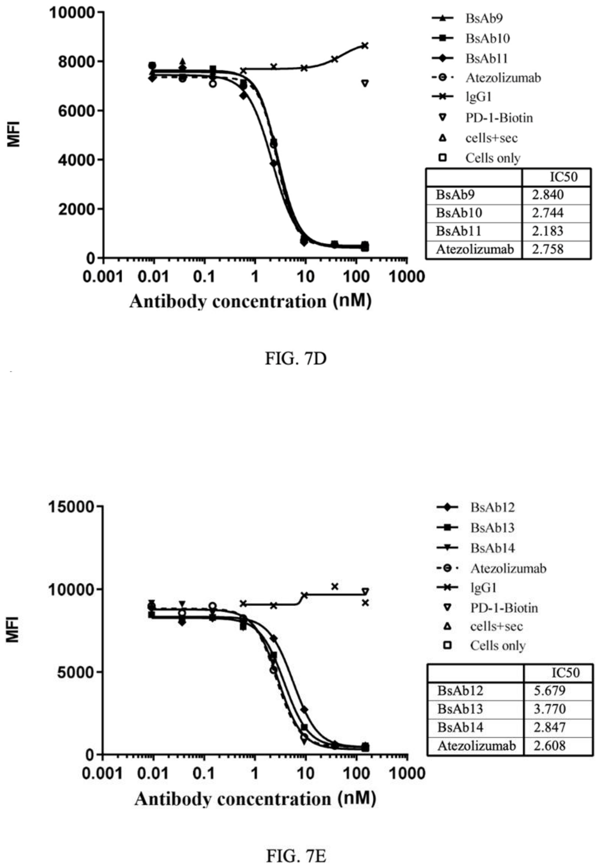

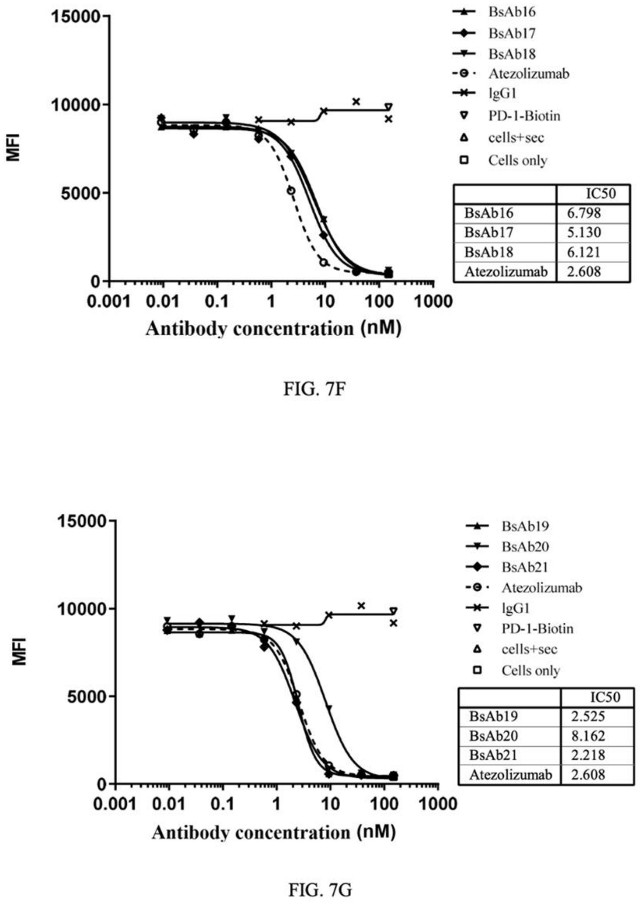

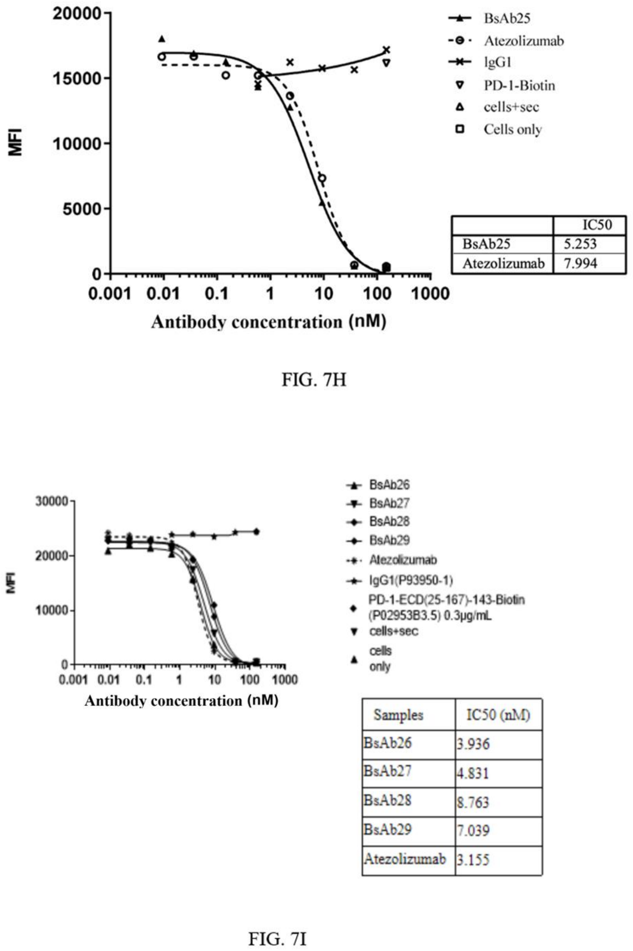

Results were shown in Figures 7A-7I. Most of the bispecific antibodies exhibited abilities comparable to the control antibody Atezolizumab in blocking the PD-1/PD-L1 binding activity. Among them, the blocking activities of BsAb4, BsAb6, BsAb11, and BsAb25 were significantly stronger than those of other candidate antibodies and the positive control antibody Atezolizumab.

### Example 7 Validation of activities of bispecific antibodies against PD-L1 and IL-1 in blocking PD-1/PD-L1 signaling pathway

Activities of the bispecific antibodies in blocking PD-1/PD-L1 signaling pathway were determined and evaluated using a luciferase reporter gene.

Specific experimental method: firstly, CD3L-huPD-L1-CHO-K and huPD-1-NF-AT-Jurkat cells were cultured and the cell with good growing condition were collected. Then, the test bispecific antibodies and the positive control Atezolizumab were serially diluted, and the diluted test antibodies were added to 96-well cell culture plates. The above two types of cells were washed and resuspended with RPMI 1640 medium, added in a ratio of 5:1 (huPD-1-NF-AT-Jurkat and CD3L-huPD-L1-CHO-K cells were 1×10⁵ and 2×10⁴ cells/well, respectively) to 96-well cell culture plates containing the test antibodies, and incubated in a cell culture incubator at 37°C for 6 h. After completion of incubation, 50 µL of the luciferase substrate Bright-Lite (Vazyme, DD1204-03) was added to each well, shaken for 60 s, and then measured for fluorescence intensities.

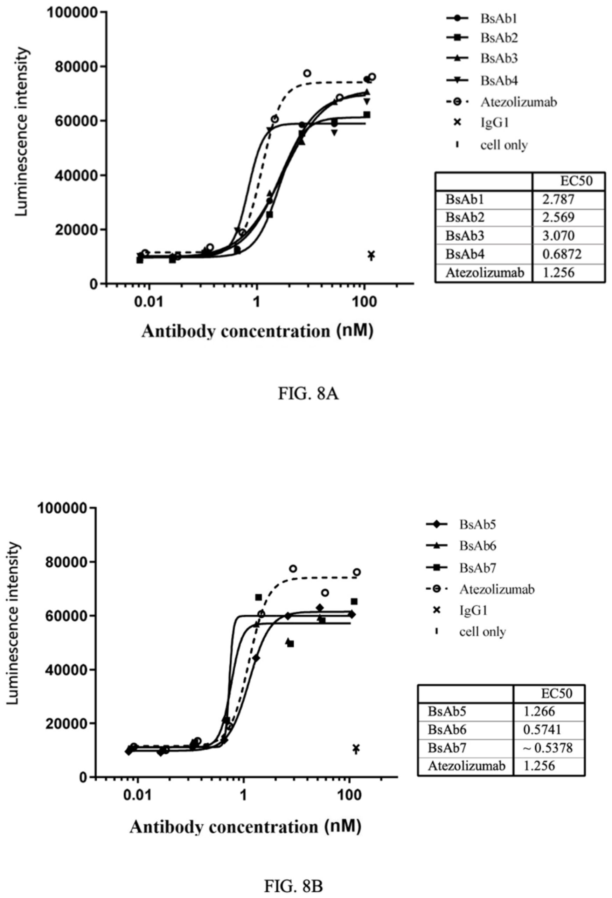

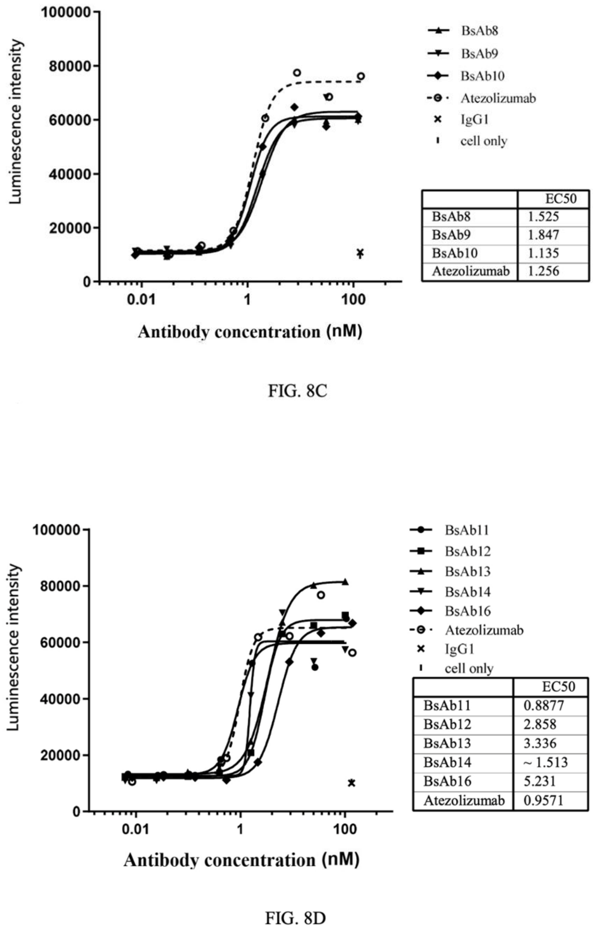

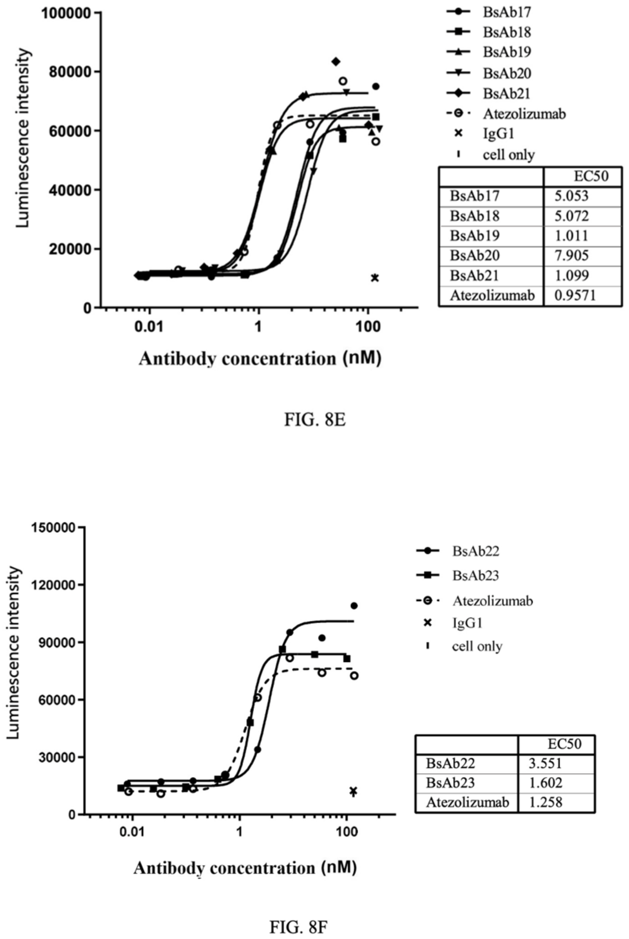

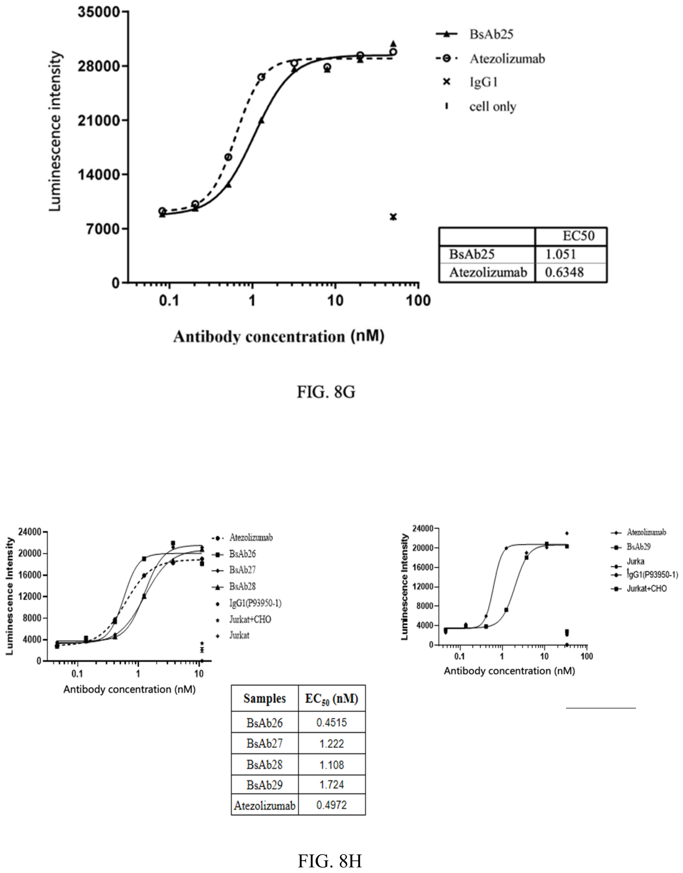

Results were shown in Figures 8A-8H. Most of the bispecific antibodies exhibited relatively strong activities in activating luciferase signaling. Among them, the activation effects of BsAb4, BsAb6, BsAb11, and BsAb26 were superior than that of the positive control antibody Atezolizumab, indicating that they had good activities in blocking the PD-1/PD-L1 signaling pathway and in immune activation.

### Example 8 Analysis of steric hindrance of preferred bispecific antibodies in binding the two antigen targets at protein level

This Example evaluated the abilities of preferred bispecific antibody molecules in binding both PD-L1 and IL-1. The specific experimental method was as follows:
96-well ELISA plates were coated with human recombinant protein huPD-L1-Fc, huIL-1R1-Fc, or huIL-1β-Fc overnight at 4°C. The next day, the plates were washed with PBST for 3 times and then blocked with 5% skim milk at room temperature for 2 h. Washing was carried out with PBST for 3 times, and then different concentrations of test antibodies were added and incubated for 1 h. Washing was carried out with PBST for 3 times, and then another antigen huIL-1R1-His or huIL-1β-His, huPD-L1-His was added and incubated at room temperature for 1 h. Thereafter, washing was carried out with PBST for 3 times, and then the secondary antibody Anti-6*His-HRP (proteintech; HRP-66005) was added and incubated at room temperature for 50 min. After completion of incubation, the plates were washed with PBST for six times and added with TMB (SurModics, TMBS-1000-01) for color development. Based on the results of the color development, 2 M HCl was added to terminate reactions, and OD450 values were read by a microplate reader (Molecular Devices, SpecterMax 190). Data were analyzed using PRISM^{™} (GraphPad Software, San Diego, CA) and EC₅₀ values (nM) were calculated.

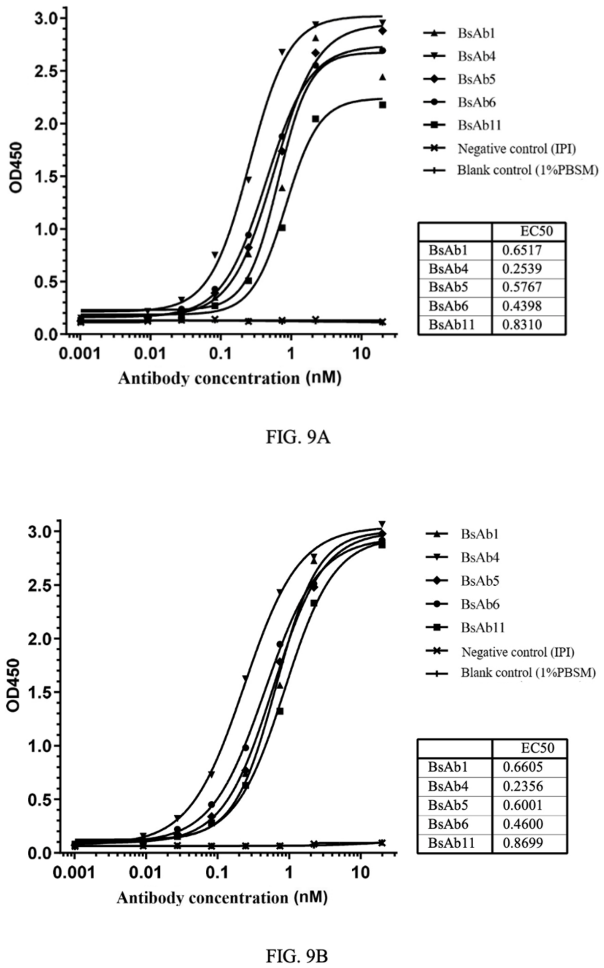

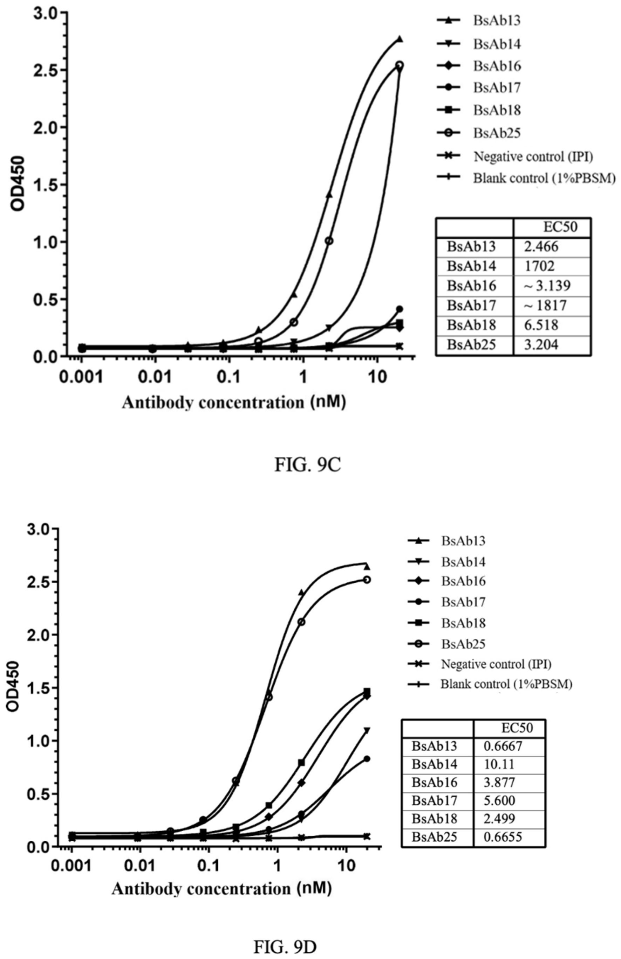

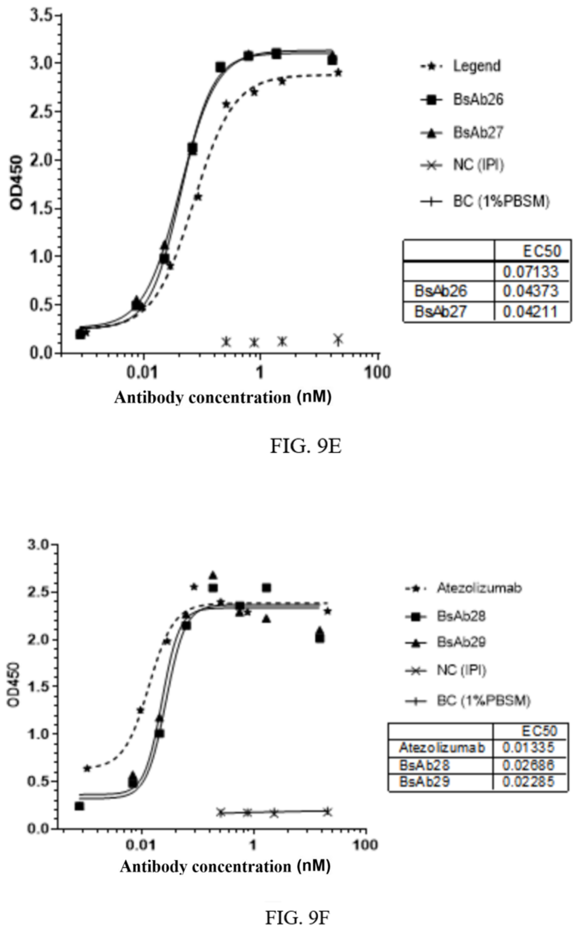

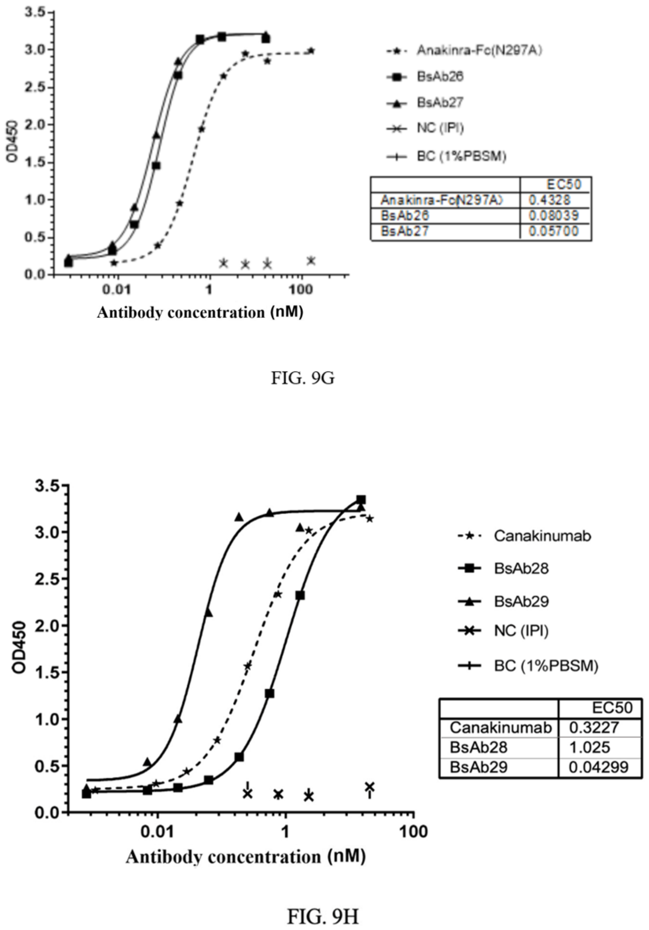

The binding abilities of the preferred bispecific antibodies to both huPD-L1 and IL-1R1 or IL-1β were shown in Figures 9A-9H (Figure 9A represented coated huPD-L1-F with IL-1R1-His added; Figure 9B represented coated huIL-1R1-Fc with huPD-L1-His added; Figure 9C represented coated huPD-L1-F with huIL-1β-His added; and Figure 9D represented coated huIL-1β-Fc with huPD-L1-His added). Among them, BsAb1, BsAb4, BsAb6, BsAb11, BsAb13, BsAb5, BsAb26, BsAb27, BsAb28, and BsAb29 bound both the PD-L1 and the IL-1R1 or IL-1β antigens with high affinities.

The amino acid sequences referred to herein are as follows.

| SEQ ID NO | Description of sequence | Information of sequence |
|---|---|---|
| 1 | Extracellular region of human IL-1β | APVRSLNCTLRDSQQKSLVMSGPYELKALHLQGQDMEQQVVFSMSFVQGEES NDKIPVALGLKEKNLYLSCVLKDDKPTLQLESVDPKNYPKKKMEKRFVFNKIE INNKLEFESAQFPNWYISTSQAENMPVFLGGTKGGQDITDFTMQFVSS |
| 2 | Extracellular region of human IL-1R1 | LEADKCKEREEKIILVSSANEIDVRPCPLNPNEHKGTITWYKDDSKTPVSTEQA SRIHQHKEKLWFVPAKVEDSGHYYCVVRNSSYCLRIKISAKFVENEPNLCYNA QAIFKQKLPVAGDGGLVCPYMEFFKNENNELPKLQWYKDCKPLLLDNIHFSG VKDRLIVMNVAEKHRGNYTCHASYTYLGKQYPITRVIEFITLEENKPTRPVIVS PANETMEVDLGSQIQLICNVTGQLSDIAYWKWNGSVIDEDDPVLGEDYYSVE NPANKRRSTLITVLNISEIESRFYKHPFTCFAKNTHGIDAAYIQLIYPVTNFQK |
| 3 | Extracellular region of human PD-L1 | FTVTVPKDLYVVEYGSNMTIECKFPVEKQLDLAALIVYWEMEDKNIIQFVHGE EDLKVQHSSYRQRARLLKDQLSLGNAALQITDVKLQDAGVYRCMISYGGAD YKRITVKVNAPYNKINQRILVVDPVTSEHELTCQAEGYPKAEVIWTSSDHQVL SGKTTTTNSKREEKLFNVTSTLRINTTTNEIFYCTFRRLDPEENHTAELVIPELPL AHPPNER |
| 4 | Fc segment of human IgG1 | EPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCS VMHEALHNHYTQKSLSLSPGK |
| 5 | His tag | HHHHHH |
| 6 | Light chain of Canakinumab | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 7 | Heavy chain of Canakinumab | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKRVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSL TCLVKGFYPSDIAVEWESNGQPENNYKTTPPVLDSDGSFFLYSKLTVDKSRWQ QGNVFSCSVMHEALHNHYTQKSLSLSPGK |
| 8 | Light chain of Atezolizumab | DIQMTQSPSSLSASVGDRVTITCRASQDVSTAVAWYQQKPGKAPKLLIYSASFL YSGVPSRFSGSGSGTDFTLTISSLQPEDFATYYCQQYLYHPATFGQGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| 9 | Heavy chain of Atezolizumab | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK |
| | | VDKKVEPKSCDKTHTCPPCPAPELLGGPSVFLFPPKPKDTLMISRTPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNV FSCSVMHEALHNHYTQKSLSLSPGK |
| 10 | Anakinra-Fc | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEEPKSCDK THTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALH NHYTQKSLSLSPGK |
| 11 | Full-length human IL-1R1 | MKVLLRLICFIALLISFLEADKCNEREEKIILVSSANEIDVRPCPLNPNEYKGTIT WYKNDSKTPISTEQASRIHQHKKKLWFVPAKVEDSGHYYCVVRNSSYCLRIKI TAKFVENEPNLCYNAEAIFKQRLPVAGDGGLVCPYMEFFKDENNELPKLLWY KDCKPLLLDNIHFSGVKDRLIVMNVAEKHRGNYTCHASYTYLGKQYPITRVIE FITLEENKPTRPVIVSPANETIEVDLGSQIQLICNVTGQLSDTAYWKWNGSFIDE DDPVLGEDYYSVENPANKRRSTLITVLNISETESRFYKHPFTCLARNTHGMDA AYVQLIYPVTKFQKHMIGICVTLTVIIMCSVFIYKIFKVDIVLWYRDSCYDFLPI KASDGKTYDAYILYPKTAGEGSTSDCDIFVFKVLPEVLEKQCGYKLFIFGRDDY VGEDIVEVINENVKKSRRLIIILVREISGFSWLGGSSEEQIAMYNALVQDGIKVV LLELEKIQDYEKMPESIKFIKRKHGVIRWSGDFTQGPQSAKTRFWKNVRYHMP VQRRSPSSKHQLPSPATKEKLQREAHVPLG |
| 12 | Full-length human PD-L1 | MRIFAVFIFMTYWHLLNAFTVTVPKDLYVVEYGSNMTIECKFPVEKQLDLAAL IVYWEMEDKNIIQFVHGEEDLKVQHSSYRQRARLLKDQLSLGNAALQITDVK LQDAGVYRCMISYGGADYKRITVKVNAPYNKINQRILVVDPVTSEHELTCQAE GYPKAEVIWTSSDHQVLSGKTTTTNSKREEKLFNVTSTLRINTTTNEIFYCTFR RLDPEENHTAELVIPELPLAHPPNERTHLVILGAILLCLGVALTFIFRLRKGRMM DVKKCGIQDTNSKKQSDTHLEET |
| 13 | Full-length human PD-1 | MQIPQAPWPVVWAVLQLGWRPGWFLDSPDRPWNPPTFSPALLVVTEGDNATF TCSFSNTSESFVLNWYRMSPSNQTDKLAAFPEDRSQPGQDCRFRVTQLPNGRD FHMSVVRARRNDSGTYLCGAISLAPKAQIKESLRAELRVTERRAEVPTAHPSPS PRPAGQFQTLVVGVVGGLLGSLVLLVWVLAVICSRAARGTIGARRTGQPLKED PSAVPVFSVDYGELDFQWREKTPEPPVPCVPEQTEYATIVFPSGMGTSSPARRG SADGPRSAQPLRPEDGHCSWPL |
| 14 | Anakinra | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDE |
| 15 | Canakinumab VH | |
| 16 | Canakinumab VL | |
| 17 | Atezolizumab VH | |
| 18 | Atezolizumab VL | |
| 19 | (G₄S)₃G | GGGGSGGGGSGGGGSG |
| 20 | (G₄S)₄G | GGGGSGGGGSGGGGSGGGGSG |
| 21 | (G₄S)₃ | GGGGSGGGGSGGGGS |
| 22 | (G₄S)₄ | GGGGSGGGGSGGGGSGGGGS |
| 23 | Linker | TVAAPSVFIFPP |
| 24 | Linker | ASTKGPSVFPLAP |
| 25 | First polypeptide chain of BsAb1, | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG |
| | BsAb4 | FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNV FSCSVMHEALHNHYTQKSLSLSPGK |
| 26 | Second polypeptide chain of BsAbl | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEGGGGSG GGGSGGGGSGGGGSGDIQMTQSPSSLSASVGDRVTITCRASQDVSTAVAWYQ QKPGKAPKLLIYSASFLYSGVPSRFSGSGSGTDFTLTISSLQPEDFATYYCQQYL YHPATFGQGTKVEIKRTVAAPSVFIFPPSDEQLKSGTASVVCLLNNFYPREAKV QWKVDNALQSGNSQESVTEQDSKDSTYSLSSTLTLSKADYEKHKVYACEVTH QGLSSPVTKSFNRGEC |
| 27 | First polypeptide chain of BsAb2 | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEGGGGSG GGGSGGGGSGGGGSGEVQLVESGGGLVQPGGSLRLSCAASGFTFSDSWIHWV RQAPGKGLEWVAWISPYGGSTYYADSVKGRFTISADTSKNTAYLQMNSLRAE DTAVYYCARRHWPGGFDYWGQGTLVTVSSASTKGPSVFPLAPSSKSTSGGTA ALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYASTY RVVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 28 | Second polypeptide chain of BsAb2, BsAb3, BsAb5, BsAb6, BsAb8, BsAb9, BsAbl5, BsAb16, BsAb17, BsAb18, BsAb22, BsAb23, BsAb27 | DIQMTQSPSSLSASVGDRVTITCRASQDVSTAVAWYQQKPGKAPKLLIYSASFL YSGVPSRFSGSGSGTDFTLTISSLQPEDFATYYCQQYLYHPATFGQGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| | Third polypeptide chain of BsAb7, BsAb10, BsAb11, BsAb19, BsAb20, BsAb21 | |
| 29 | First polypeptide chain of BsAb3 | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEGGGGSG GGGSGGGGSGEVQLVESGGGLVQPGGSLRLSCAASGFTFSDSWIHWVRQAPG KGLEWVAWISPYGGSTYYADSVKGRFTISADTSKNTAYLQMNSLRAEDTAVY YCARRHWPGGFDYWGQGTLVTVSSASTKGPSVFPLAPSSKSTSGGTAALGCL VKDYFPEPVTVSWNSGALTSGVHTFPAVLQSSGLYSLSSVVTVPSSSLGTQTYI CNVNHKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYASTYRVVS VLTVLHQDWLNGKEYKCKVSNKALPAPIEKTISKAKGQPREPQVYTLPPSREE MTKNQVSLTCLVKGFYPSDIAVEWESNGQPENNYKTTPPVLDSDGSFFLYSKL TVDKSRWQQGNVFSCSVMHEALHNHYTQKSLSLSPGK |
| 30 | Second polypeptide chain of BsAb4, BsAb26 | DIQMTQSPSSLSASVGDRVTITCRASQDVSTAVAWYQQKPGKAPKLLIYSASFL YSGVPSRFSGSGSGTDFTLTISSLQPEDFATYYCQQYLYHPATFGQGTKVEIKRT VAAPSVFIFPPSDEQLKSGTASVVCLLNNFYPREAKVQWKVDNALQSGNSQES V |
| | | TEQDSKDSTYSLSSTLTLSKADYEKHKVYACEVTHQGLSSPVTKSFNRGECGG GGSGGGGSGGGGSGGGGSGRPSGRKSSKMQAFRIWDVNQKTFYLRNNQLVA GYLQGPNVNLEEKIDVVPIEPHALFLGIHGGKMCLSCVKSGDETRLQLEAVNIT DLSENRKQDKRFAFIRSDSGPTTSFESAACPGWFLCTAMEADQPVSLTNMPDE GVMVTKFYFQEDE |
| 31 | First polypeptide chain of BsAb5 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNV FSCSVMHEALHNHYTQKSLSLSPGKGGGGSGGGGSGGGGSGGGGSGRPSGR KSSKMQAFRIWDVNQKTFYLRNNQLVAGYLQGPNVNLEEKIDVVPIEPHALFL GIHGGKMCLSCVKSGDETRLQLEAVNITDLSENRKQDKRFAFIRSDSGPTTSFE SAACPGWFLCTAMEADQPVSLTNMPDEGVMVTKFYFQEDE |
| 32 | First polypeptide chain of BsAb6 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNV FSCSVMHEALHNHYTQKSLSLSPGKGGGGSGGGGSGGGGSGRPSGRKSSKM QAFRIWDVNQKTFYLRNNQLVAGYLQGPNVNLEEKIDVVPIEPHALFLGIHGG KMCLSCVKSGDETRLQLEAVNITDLSENRKQDKRFAFIRSDSGPTTSFESAACP GWFLCTAMEADQPVSLTNMPDEGVMVTKFYFQEDE |
| 33 | First polypeptide chain of BsAb7, BsAb8, BsAb9, BsAb10, BsAbl6, BsAbl7, BsAb18, BsAb20 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPCRDELTKNQVSLWCL VKGFYPSDIAVEWESNGQPENNYKTTPPVLDSDGSFFLYSKLTVDKSRWQQGN VFSCSVMHEALHNHYTQKSLSLSPGK |
| 34 | Second polypeptide chain of BsAb7 | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEGGGGSG GGGSGGGGSGGGGSGEVQLVESGGGLVQPGGSLRLSCAASGFTFSDSWIHWV RQAPGKGLEWVAWISPYGGSTYYADSVKGRFTISADTSKNTAYLQMNSLRAE DTAVYYCARRHWPGGFDYWGQGTLVTVSSASTKGPSVFPLAPSSKSTSGGTA ALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVCTLP PSRDELTKNQVSLSCAVKGFYPSDIAVEWESNGQPENNYKTTPPVLDSDGSFFL VSKLTVDKSRWQQGNVFSCSVMHEALHNHYTQKSLSLSPGK |
| 35 | Third polypeptide chain of BsAb8 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEVTH QGLSSPVTKSFNRGECDKTHTCPPCPAPELLGGPSVFLFPPKPKDTLMISRTPEV TCVVVDVSHEDPEVKFNWYVDGVEVHNAKTKPREEQYNSTYRVVSVLTVLH QDWLNGKEYKCKVSNKALPAPIEKTISKAKGQPREPQVCTLPPSRDELTKNQV |
| | | SLSCAVKGFYPSDIAVEWESNGQPENNYKTTPPVLDSDGSFFLVSKLTVDKSR WQQGNVFSCSVMHEALHNHYTQKSLSLSPGK |
| 36 | Fourth polypeptide chain of BsAb8 | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEGGGGSG GGGSGGGGSGGGGSGDIQMTQSPSSLSASVGDRVTITCRASQDVSTAVAWYQ QKPGKAPKLLIYSASFLYSGVPSRFSGSGSGTDFTLTISSLQPEDFATYYCQQYL YHPATFGQGTKVEIKSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKVEPKSC |
| 37 | Third polypeptide chain of BsAb9 | DIQMTQSPSSLSASVGDRVTITCRASQDVSTAVAWYQQKPGKAPKLLIYSASFL YSGVPSRFSGSGSGTDFTLTISSLQPEDFATYYCQQYLYHPATFGQGTKVEIKSS ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALP APIEKTISKAKGQPREPQVCTLPPSRDELTKNQVSLSCAVKGFYPSDIAVEWES NGQPENNYKTTPPVLDSDGSFFLVSKLTVDKSRWQQGNVFSCSVMHEALHNH YTQKSLSLSPGK |
| 38 | Fourth polypeptide chain of BsAb9 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEVTH QGLSSPVTKSFNRGECGGGGSGGGGSGGGGSGGGGSGRPSGRKSSKMQAFRI WDVNQKTFYLRNNQLVAGYLQGPNVNLEEKIDVVPIEPHALFLGIHGGKMCL SCVKSGDETRLQLEAVNITDLSENRKQDKRFAFIRSDSGPTTSFESAACPGWFL CTAMEADQPVSLTNMPDEGVMVTKFYFQEDE |
| 39 | Second polypeptide chain of BsAb10, BsAbll | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVCTLPPSRDELTKNQVSLSCAV KGFYPSDIAVEWESNGQPENNYKTTPPVLDSDGSFFLVSKLTVDKSRWQQGNV FSCSVMHEALHNHYTQKSLSLSPGKGGGGSGGGGSGGGGSGGGGSGRPSGR KSSKMQAFRIWDVNQKTFYLRNNQLVAGYLQGPNVNLEEKIDVVPIEPHALFL GIHGGKMCLSCVKSGDETRLQLEAVNITDLSENRKQDKRFAFIRSDSGPTTSFE SAACPGWFLCTAMEADQPVSLTNMPDEGVMVTKFYFQEDE |
| 40 | First polypeptide chain of BsAb11, BsAb21 | NTAYLQMNSLRAEDTAVYYCARRHWPGGFDYWGQGTLVTVSSASTKGPSVF PLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKA KGQPREPQVYTLPPCRDELTKNQVSLWCLVKGFYPSDIAVEWESNGQPENNY KTTPPVLDSDGSFFLYSKLTVDKSRWQQGNVFSCSVMHEALHNHYTQKSLSLS PGKGGGGSGGGGSGGGGSEVQLVESGGGLVQPGGSLRLSCAASGFTFSDSWI HWVRQAPGKGLEWVAWISPYGGSTYYADSVKGRFTISADTSKNTAYLQMNSL RAEDTAVYYCARRHWPGGFDYWGQGTLVTVSSASTKGPSVFPLAPSSKSTSG GTAALGCLVKDYFPEPVTVSWNSGALTSGVHTFPAVLQSSGLYSLSSVVTVPSS SLGTQTYICNVNHKPSNTKVDKKVEPKSC |
| 41 | First polypeptide chain of BsAb12 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSGGGGSGGGGSGGGGSEVQLVESGGGLVQPGGSLRLS CAASGFTFSDSWIHWVRQAPGKGLEWVAWISPYGGSTYYADSVKGRFTISAD TSKNTAYLQMNSLRAEDTAVYYCARRHWPGGFDYWGQGTLVTVSSASTKGP SVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEV HNAKTKPREEQYASTYRVVSVLTVLHQDWLNGKEYKCKVSNKALPAPIEKTIS KAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLS LSPGK |
| 42 | Second polypeptide chain of BsAb12 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKGG GGSGGGGSGGGGSDIQMTQSPSSLSASVGDRVTITCRASQDVSTAVAWYQQKP GKAPKLLIYSASFLYSGVPSRFSGSGSGTDFTLTISSLQPEDFATYYCQQYLYHP ATFGQGTKVEIKRTVAAPSVFIFPPSDEQLKSGTASVVCLLNNFYPREAKVQWK VDNALQSGNSQESVTEQDSKDSTYSLSSTLTLSKADYEKHKVYACEVTHQGLS SPVTKSFNRGEC |
| 43 | First polypeptide chain of BsAb13 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASTKGPSVFPLAPEVQLVESGGGLVQPGGSLRLSCAA SGFTFSDSWIHWVRQAPGKGLEWVAWISPYGGSTYYADSVKGRFTISADTSKN TAYLQMNSLRAEDTAVYYCARRHWPGGFDYWGQGTLVTVSSASTKGPSVFPL APSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAK TKPREEQYASTYRVVSVLTVLHQDWLNGKEYKCKVSNKALPAPIEKTISKAKG QPREPQVYTLPPSREEMTKNQVSLTCLVKGFYPSDIAVEWESNGQPENNYKTT PPVLDSDGSFFLYSKLTVDKSRWQQGNVFSCSVMHEALHNHYTQKSLSLSPG K |
| 44 | Second polypeptide chain of BsAb13 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKTVA APSVFIFPPDIQMTQSPSSLSASVGDRVTITCRASQDVSTAVAWYQQKPGKAPK LLIYSASFLYSGVPSRFSGSGSGTDFTLTISSLQPEDFATYYCQQYLYHPATFGQG TKVEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTK SFNRGEC |
| 45 | First polypeptide chain of BsAb14 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSGGGGSGGGGSGGGGSQVQLVESGGGVVQPGRSLRLSC AASGFTFSVYGMNWVRQAPGKGLEWVAIIWYDGDNQYYADSVKGRFTISRD NSKNTLYLQMNGLRAEDTAVYYCARDLRTGPFDYWGQGTLVTVSSASTKGPS VFPLAPSSKSTSGGTAALGCLVKDYFPEPVTVSWNSGALTSGVHTFPAVLQSSG LYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSLS PGK |
| 46 | Second polypeptide chain of BsAbl4, BsAb28 | DIQMTQSPSSLSASVGDRVTITCRASQDVSTAVAWYQQKPGKAPKLLIYSASFL YSGVPSRFSGSGSGTDFTLTISSLQPEDFATYYCQQYLYHPATFGQGTKVEIKGG GGSGGGGSGGGGSEIVLTQSPDFQSVTPKEKVTITCRASQSIGSSLHWYQQKP DQSPKLLIKYASQSFSGVPSRFSGSGSGTDFTLTINSLEAEDAAAYYCHQSSSLP FTFGPGTKVDIKRTVAAPSVFIFPPSDEQLKSGTASVVCLLNNFYPREAKVQWK VDNALQSGNSQESVTEQDSKDSTYSLSSTLTLSKADYEKHKVYACEVTHQGLS SPVTKSFNRGEC |
| 47 | First polypeptide chain of BsAb15 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNV FSCSVMHEALHNHYTQKSLSLSPGKGGGGSGGGGSGGGGSQVQLVESGGGV VQPGRSLRLSCAASGFTFSVYGMNWVRQAPGKGLEWVAIIWYDGDNQYYAD SVKGRFTISRDNSKNTLYLQMNGLRAEDTAVYYCARDLRTGPFDYWGQGTLV TVSSGGGGSGGGGSGGGGSEIVLTQSPDFQSVTPKEKVTITCRASQSIGSSLHW YQQKPDQSPKLLIKYASQSFSGVPSRFSGSGSGTDFTLTINSLEAEDAAAYYCH QSSSLPFTFGPGTKVDIK |
| 48 | Third polypeptide chain of BsAb16 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGECDK THTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVCTLPPSRDELTKNQVSLSCAVKGFYPSDIAVEW ESNGQPENNYKTTPPVLDSDGSFFLVSKLTVDKSRWQQGNVFSCSVMHEALH NHYTQKSLSLSPGK |
| 49 | Fourth polypeptide chain of BsAb16 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKKVEPKSC |
| 50 | Third polypeptide chain of BsAb17 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASVAAPSVFIFPPSDEQLKSGTASVVCLLNNFYPREAK VQWKVDNALQSGNSQESVTEQDSKDSTYSLSSTLTLSKADYEKHKVYACEVT HQGLSSPVTKSFNRGECDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVCTLPPSRDELTKNQ VSLSCAVKGFYPSDIAVEWESNGQPENNYKTTPPVLDSDGSFFLVSKLTVDKSR WQQGNVFSCSVMHEALHNHYTQKSLSLSPGK |
| 51 | Fourth polypeptide chain of BsAb17 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKSSA STKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSC |
| 52 | Third polypeptide chain of BsAb18 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKSSA STKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPA PIEKTISKAKGQPREPQVCTLPPSRDELTKNQVSLSCAVKGFYPSDIAVEWESN GQPENNYKTTPPVLDSDGSFFLVSKLTVDKSRWQQGNVFSCSVMHEALHNHY TQKSLSLSPGK |
| 53 | Fourth polypeptide chain of BsAb18 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASVAAPSVFIFPPSDEQLKSGTASVVCLLNNFYPREAK VQWKVDNALQSGNSQESVTEQDSKDSTYSLSSTLTLSKADYEKHKVYACEVT HQGLSSPVTKSFNRGEC |
| 54 | First polypeptide chain of BsAb19 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPCRDELTKNQVSLWCL VKGFYPSDIAVEWESNGQPENNYKTTPPVLDSDGSFFLYSKLTVDKSRWQQGN VFSCSVMHEALHNHYTQKSLSLSPGKGGGGSGGGGSGGGGSQVQLVESGGG VVQPGRSLRLSCAASGFTFSVYGMNWVRQAPGKGLEWVAIIWYDGDNQYYA DSVKGRFTISRDNSKNTLYLQMNGLRAEDTAVYYCARDLRTGPFDYWGQGTL VTVSSGGGGSGGGGSGGGGSEIVLTQSPDFQSVTPKEKVTITCRASQSIGSSLH WYQQKPDQSPKLLIKYASQSFSGVPSRFSGSGSGTDFTLTINSLEAEDAAAYYC HQSSSLPFTFGPGTKVDIK |
| 55 | Second polypeptide chain of BsAb19 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVCTLPPSRDELTKNQVSLSCAV KGFYPSDIAVEWESNGQPENNYKTTPPVLDSDGSFFLVSKLTVDKSRWQQGNV FSCSVMHEALHNHYTQKSLSLSPGK |
| 56 | Second polypeptide chain of BsAb20 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSGGGGSGGGGSGGGGSEIVLTQSPDFQSVTPKEKVTIT CRASQSIGSSLHWYQQKPDQSPKLLIKYASQSFSGVPSRFSGSGSGTDFTLTINS LEAEDAAAYYCHQSSSLPFTFGPGTKVDIKGGGGSGGGGSGGGGSDKTHTCP PCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPI EKTISKAKGQPREPQVCTLPPSRDELTKNQVSLSCAVKGFYPSDIAVEWESNGQ PENNYKTTPPVLDSDGSFFLVSKLTVDKSRWQQGNVFSCSVMHEALHNHYTQ KSLSLSPGK |
| 57 | Second polypeptide chain of BsAb21 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVCTLPPSRDELTKNQVSLSCAV KGFYPSDIAVEWESNGQPENNYKTTPPVLDSDGSFFLVSKLTVDKSRWQQGNV FSCSVMHEALHNHYTQKSLSLSPGKGGGGSGGGGSGGGGSQVQLVESGGGV VQPGRSLRLSCAASGFTFSVYGMNWVRQAPGKGLEWVAIIWYDGDNQYYAD SVKGRFTISRDNSKNTLYLQMNGLRAEDTAVYYCARDLRTGPFDYWGQGTLV TVSSGGGGSGGGGSGGGGSEIVLTQSPDFQSVTPKEKVTITCRASQSIGSSLHW YQQKPDQSPKLLIKYASQSFSGVPSRFSGSGSGTDFTLTINSLEAEDAAAYYCH QSSSLPFTFGPGTKVDIK |
| 58 | First polypeptide chain of BsAb22 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFLLYSKLTVDKSRWQQGNV FSCSVMHEALHNHYTQKSLSLSPGK |
| 59 | Third polypeptide chain of BsAb22 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASVAAPSVFIFPPSDEQLKSGTASVVCLLNNFYPREAK VQWKVDNALQSGNSQESVTEQDSKDSTYSLSSTLTLSKADYEKHKVYACEVT HQGLSSPVTKSFNRGECDKTHTCPPCPAPELLGGPSVFLFPPKPKDTLMISRTPE VTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKN QVSLTCLVKGFYPSDIAVEWESNGQPENNYKTTPPVLDSDGSFFLYSRLTVDKS RWQQGNVFSCSVMHEALHNHYTQKSLSLSPGK |
| 60 | Fourth polypeptide chain of BsAb22 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKSSA STKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKRVEPKSC |
| 61 | First polypeptide chain of BsAb23 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNV FSCSVMHEALHNHYTQKSLSLSPGKGGGGSGGGGSGGGGSGGGGSQVQLVE SGGGVVQPGRSLRLSCAASGFTFSVYGMNWVRQAPGKGLEWVAIIWYDGDN QYYADSVKGRFTISRDNSKNTLYLQMNGLRAEDTAVYYCARDLRTGPFDYWG QGTLVTVSSGGGGSGGGGSGGGGSGGGGSEIVLTQSPDFQSVTPKEKVTITCR ASQSIGSSLHWYQQKPDQSPKLLIKYASQSFSGVPSRFSGSGSGTDFTLTINSLE AEDAAAYYCHQSSSLPFTFGPGTKVDIK |
| 62 | First polypeptide chain of BsAb25 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKRVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQVSL TCLVKGFYPSDIAVEWESNGQPENNYKTTPPVLDSDGSFFLYSKLTVDKSRWQ QGNVFSCSVMHEALHNHYTQKSLSLSPGKGGGGSGGGGSGGGGSGGGGSEV QLVESGGGLVQPGGSLRLSCAASGFTFSDSWIHWVRQAPGKCLEWVAWISPY GGSTYYADSVKGRFTISADTSKNTAYLQMNSLRAEDTAVYYCARRHWPGGFD YWGQGTLVTVSSGGGGSGGGGSGGGGSGGGGSDIQMTQSPSSLSASVGDRVT ITCRASQDVSTAVAWYQQKPGKAPKLLIYSASFLYSGVPSRFSGSGSGTDFTLTI SSLQPEDFATYYCQQYLYHPATFGCGTKVEIK |
| 63 | Second polypeptide chain of BsAb25, BsAb29 | EIVLTQSPDFQSVTPKEKVTITCRASQSIGSSLHWYQQKPDQSPKLLIKYASQSF SGVPSRFSGSGSGTDFTLTINSLEAEDAAAYYCHQSSSLPFTFGPGTKVDIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 64 | Canakinumab scFv | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSEIVLTQSPDFQSVTPKEKVTITCRASQSIGSSLHWYQQ KPDQSPKLLIKYASQSFSGVPSRFSGSGSGTDFTLTINSLEAEDAAAYYCHQSSS LPFTFGPGTKVDIK |
| 65 | Atezolizumab scFv | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSDIQMTQSPSSLSASVGDRVTITCRASQDVSTAVAWYQQK PGKAPKLLIYSASFLYSGVPSRFSGSGSGTDFTLTISSLQPEDFATYYCQQYLYHP ATFGQGTKVEIK |
| 66 | Anakinra-Fc (N297A) | RPSGRKSSKMQAFRIWDVNQKTFYLRNNQLVAGYLQGPNVNLEEKIDVVPIEP HALFLGIHGGKMCLSCVKSGDETRLQLEAVNITDLSENRKQDKRFAFIRSDSGP TTSFESAACPGWFLCTAMEADQPVSLTNMPDEGVMVTKFYFQEDEEPKSSDK THTCPPCPAPELLGGPSVFLFPPKPKDTLMISRTPEVTCVVVDVSHEDPEVKFN WYVDGVEVHNAKTKPREEQYASTYRVVSVLTVLHQDWLNGKEYKCKVSNK ALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALH NHYTQKSLSLSPGK |
| 67 | Fc segment of human IgG1 (N297A) | EPKSSDKTHTCPPCPAPELLGGPSVFLFPPKPKDTLMISRTPEVTCVVVDVSHE DPEVKFNWYVDGVEVHNAKTKPREEQYASTYRVVSVLTVLHQDWLNGKEY KCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCS VMHEALHNHYTQKSLSLSPGK |
| 68 | Heavy chain constant region of human IgG1 | ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHT CPPCPAPELLGGPSVFLFPPKPKDTLMISRTPEVTCVVVDVSHEDPEVKFNWYV DGVEVHNAKTKPREEQYASTYRVVSVLTVLHQDWLNGKEYKCKVSNKALPA PIEKTISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEALHNHY TQKSLSLSPGK |
| 69 | First polypeptide chain of BsAb26 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNV FSCSVMHEALHNHYTQKSLSLSPGK |
| 70 | First polypeptide chain of BsAb27 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVV DVSHEDPEVKFNWYVDGVEVHNAKTKPREEQYASTYRVVSVLTVLHQDWLN GKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNV FSCSVMHEALHNHYTQKSLSLSPGKGGGGSGGGGSGGGGSGRPSGRKSSKM QAFRIWDVNQKTFYLRNNQLVAGYLQGPNVNLEEKIDVVPIEPHALFLGIHGG KMCLSCVKSGDETRLQLEAVNITDLSENRKQDKRFAFIRSDSGPTTSFESAACP GWFLCTAMEADQPVSLTNMPDEGVMVTKFYFQEDE |
| 71 | First polypeptide chain of BsAb28 | EVQLVESGGGLVQPGGSLRLSCAASGFTFSDSWIHWVRQAPGKGLEWVAWIS PYGGSTYYADSVKGRFTISADTSKNTAYLQMNSLRAEDTAVYYCARRHWPGG FDYWGQGTLVTVSSGGGGSGGGGSGGGGSQVQLVESGGGVVQPGRSLRLSC AASGFTFSVYGMNWVRQAPGKGLEWVAIIWYDGDNQYYADSVKGRFTISRD NSKNTLYLQMNGLRAEDTAVYYCARDLRTGPFDYWGQGTLVTVSSASTKGPS VFPLAPSSKSTSGGTAALGCLVKDYFPEPVTVSWNSGALTSGVHTFPAVLQSSG LYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVH NAKTKPREEQYASTYRVVSVLTVLHQDWLNGKEYKCKVSNKALPAPIEKTISK AKGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSLS PGK |
| 72 | First polypeptide chain of BsAb29 | QVQLVESGGGVVQPGRSLRLSCAASGFTFSVYGMNWVRQAPGKGLEWVAII WYDGDNQYYADSVKGRFTISRDNSKNTLYLQMNGLRAEDTAVYYCARDLRT GPFDYWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSN TKVDKRVEPKSCDKTHTCPPCPAPELLGGPSVFLFPPKPKDTLMISRTPEVTCV VVDVSHEDPEVKFNWYVDGVEVHNAKTKPREEQYASTYRVVSVLTVLHQDW LNGKEYKCKVSNKALPAPIEKTISKAKGQPREPQVYTLPPSREEMTKNQVSLT CLVKGFYPSDIAVEWESNGQPENNYKTTPPVLDSDGSFFLYSKLTVDKSRWQQ GNVFSCSVMHEALHNHYTQKSLSLSPGKGGGGSGGGGSGGGGSGGGGSEVQ LVESGGGLVQPGGSLRLSCAASGFTFSDSWIHWVRQAPGKCLEWVAWISPYG GSTYYADSVKGRFTISADTSKNTAYLQMNSLRAEDTAVYYCARRHWPGGFDY WGQGTLVTVSSGGGGSGGGGSGGGGSGGGGSDIQMTQSPSSLSASVGDRVTI TCRASQDVSTAVAWYQQKPGKAPKLLIYSASFLYSGVPSRFSGSGSGTDFTLTIS SLQPEDFATYYCQQYLYHPATFGCGTKVEIK |

## Claims

1. A bispecific antibody comprising a first moiety targeting PD-L1 and a second moiety targeting IL-1, wherein the first moiety comprises an anti-PD-L1 antibody or an antigen-binding fragment thereof, and the anti-PD-L1 antibody comprises an HCDR1, an HCDR2, and an HCDR3 from a heavy chain variable region (VH) shown in SEQ ID NO: 17, and an LCDR1, an LCDR2, and an LCDR3 from a light chain variable region (VL) shown in SEQ ID NO: 18.

2. The bispecific antibody of claim 1, wherein the anti-PD-L1 antibody comprises a sequence shown in SEQ ID NO: 17 or an amino acid sequence having at least 90% sequence identity therewith, and a sequence shown in SEQ ID NO: 18 or an amino acid sequence having at least 90% sequence identity therewith.

3. The bispecific antibody of claim 1 or 2, wherein a heavy chain constant region of the anti-PD-L1 antibody is from an IgG1, and a light chain constant region of the anti-PD-L1 antibody is from a κ light chain.

4. The bispecific antibody of any one of claims 1-3, wherein the second moiety comprises an extracellular binding domain of an IL-1 receptor (IL-1R) or a variant thereof.

5. The bispecific antibody of any one of claims 1-4, wherein the second moiety comprises a sequence shown in SEQ ID NO: 14 or an amino acid sequence having at least 90% sequence identity therewith.

6. The bispecific antibody of any one of claims 1-5, wherein the number of the second moiety is two, and the second moieties are respectively connected at an N-terminus of the VH of the anti-PD-L1 antibody, an N-terminus of the VL of the anti-PD-L1 antibody, a C-terminus of the heavy chain constant region of the anti-PD-L1 antibody, or a C-terminus of the light chain constant region of the anti-PD-L1 antibody via an identical or different linker sequence.

7. The bispecific antibody of any one of claims 1-6, wherein the number of the second moiety is one, and the second moiety is connected at an N-terminus of the VH of the anti-PD-L1 antibody, an N-terminus of the VL of the anti-PD-L1 antibody, a C-terminus of the heavy chain constant region of the anti-PD-L1 antibody, or a C-terminus of the light chain constant region of the anti-PD-L1 antibody via a linker sequence.

8. The bispecific antibody of any one of claims 1-7, wherein the first moiety further has an antigen-binding fragment of the anti-PD-L1 antibody in an Fab format connected via a linker sequence to a C-terminus of the heavy chain constant region of the anti-PD-L1 antibody.

9. The bispecific antibody of any one of claims 1-8, wherein the second moiety is an anti-IL-1β antibody or an antigen-binding fragment thereof.

10. The bispecific antibody of any one of claims 1-9, wherein the anti-IL-1β antibody comprises an HCDR1, an HCDR2, and an HCDR3 from a VH shown in SEQ ID NO: 15, and an LCDR1, an LCDR2, and an LCDR3 from a VL shown in SEQ ID NO: 16.

11. The bispecific antibody of any one of claims 1-10, wherein the anti-IL-1β antibody comprises a sequence shown in SEQ ID NO: 15 or an amino acid sequence having at least 90% sequence identity therewith, and a sequence shown in SEQ ID NO: 16 or an amino acid sequence having at least 90% sequence identity therewith.

12. The bispecific antibody of any one of claims 1-11, wherein a heavy chain constant region of the anti-IL-1β antibody is from an IgG1, and a light chain constant region of the anti-PD-L1 antibody is from a κ light chain.

13. The bispecific antibody of any one of claims 1-12, wherein the antigen-binding fragment of the anti-IL-1β antibody is in an Fab or scFv format.

14. The bispecific antibody of any one of claims 1-13, wherein a heavy chain constant region of the anti-PD-L1 antibody and/or a heavy chain constant region of the anti-IL-1β antibody are/is mutated so that a Knob-hole binding is capable of being formed between the heavy chain constant regions.

15. The bispecific antibody of any one of claims 1-14, wherein the first moiety and the second moiety are connected via an identical or different linker sequence, and preferably, the linker sequence is selected from (G₄S)₄G, (G₄S)₃G, (G₄S)₄ and (G₄S)₃.

16. The bispecific antibody of any one of claims 1-15, wherein a heavy chain of the anti-PD-L1 antibody comprises a sequence shown in SEQ ID NO: 9 or an amino acid sequence having at least 90% sequence identity therewith, and a light chain of the anti-PD-L1 antibody comprises a sequence shown in SEQ ID NO: 8 or an amino acid sequence having at least 90% sequence identity therewith.

17. The bispecific antibody of any one of claims 1-16, wherein a heavy chain of the anti-IL-1β antibody comprises a sequence shown in SEQ ID NO: 7 or an amino acid sequence having at least 90% sequence identity therewith, and a light chain of the anti-IL-1β antibody comprises a sequence shown in SEQ ID NO: 6 or an amino acid sequence having at least 90% sequence identity therewith.

18. The bispecific antibody of any one of claims 1-17, wherein the antigen-binding fragment of the anti-PD-L1 antibody in an scFv format comprises a sequence shown in SEQ ID NO: 65 or an amino acid sequence having at least 90% sequence identity therewith.

19. The bispecific antibody of any one of claims 1-18, wherein an antigen-binding fragment of the anti-IL-1β antibody in an scFv format comprises a sequence shown in SEQ ID NO: 64 or an amino acid sequence having at least 90% sequence identity therewith.

20. The bispecific antibody of any one of claims 1-19, having any of the schematic antibody structures in Figures 1A-1I.

21. The bispecific antibody of any one of claims 1-19, having any of the schematic antibody structures in Figures 2A-2J.

22. The bispecific antibody of any one of claims 1-21, wherein the extracellular binding domain of the IL-1 receptor (IL-1R) or a variant thereof is at its C-terminus connected to an Fc fragment.

23. The bispecific antibody of any one of claims 1-22, wherein the heavy chain constant region and/or the Fc fragment comprise(s) an N297A mutation.

24. The bispecific antibody of any one of claims 1-23, wherein the heavy chain constant region and/or the Fc fragment are/is from a human IgG1.

25. The bispecific antibody of any one of claims 1-24, wherein the heavy chain constant region comprises a sequence shown in SEQ ID NO: 68, and the Fc fragment comprises a sequence shown in SEQ ID NO: 67.

26. The bispecific antibody of any one of claims 1-25, wherein the second moiety comprises a sequence shown in SEQ ID NO: 10 or 66.

27. The bispecific antibody of any one of claims 1-26, wherein the bispecific antibody comprises a polypeptide chain listed in any row of Table 1.

28. The bispecific antibody of any one of claims 1-27, wherein the bispecific antibody comprises:
1) a polypeptide chain with a sequence shown in SEQ ID NO: 25 and a polypeptide chain with a sequence shown in SEQ ID NO: 26;
2) a polypeptide chain with a sequence shown in SEQ ID NO: 25 and a polypeptide chain with a sequence shown in SEQ ID NO: 30;
3) a polypeptide chain with a sequence shown in SEQ ID NO: 32 and a polypeptide chain with a sequence shown in SEQ ID NO: 28;
4) a polypeptide chain with a sequence shown in SEQ ID NO: 40, a polypeptide chain with a sequence shown in SEQ ID NO: 39, and a polypeptide chain with a sequence shown in SEQ ID NO: 28;
5) a polypeptide chain with a sequence shown in SEQ ID NO: 43 and a polypeptide chain with a sequence shown in SEQ ID NO: 44;
6) a polypeptide chain with a sequence shown in SEQ ID NO: 45 and a polypeptide chain with a sequence shown in SEQ ID NO: 46;
7) a polypeptide chain with a sequence shown in SEQ ID NO: 33, a polypeptide chain with a sequence shown in SEQ ID NO: 28, a polypeptide chain with a sequence shown in SEQ ID NO: 50, and a polypeptide chain with a sequence shown in SEQ ID NO: 51; or
8) a polypeptide chain with a sequence shown in SEQ ID NO: 62 and a polypeptide chain with a sequence shown in SEQ ID NO: 63;
9) a polypeptide chain with a sequence shown in SEQ ID NO: 69 and a polypeptide chain with a sequence shown in SEQ ID NO: 30;
10) a polypeptide chain with a sequence shown in SEQ ID NO: 70 and a polypeptide chain with a sequence shown in SEQ ID NO: 28;
11) a polypeptide chain with a sequence shown in SEQ ID NO: 71 and a polypeptide chain with a sequence shown in SEQ ID NO: 46; or
12) a polypeptide chain with a sequence shown in SEQ ID NO: 72 and a polypeptide chain with a sequence shown in SEQ ID NO: 63.

29. A nucleic acid molecule encoding the bispecific antibody of any one of claims 1-28 or a polypeptide chain thereof.

30. An expression vector comprising the nucleic acid molecule of claim 29.

31. A pharmaceutical composition comprising:
1) the bispecific antibody of any one of claims 1-28, the nucleic acid molecule of claim 29, or the expression vector of claim 30; and
2) a pharmaceutically acceptable carrier.

32. Use of the bispecific antibody of any one of claims 1-28, the nucleic acid molecule of claim 29, or the expression vector of claim 30 in the manufacture of a medicament for treating a tumor.

33. The use of claim 32, wherein the tumor is selected from malignant melanoma, non-small cell lung cancer, renal cell carcinoma, bladder cancer, gastric cancer, breast cancer, pancreatic cancer, and lung cancer.

34. A method of treating a tumor in a subject, comprising administering to the subject a therapeutically effective amount of the bispecific antibody of any one of claims 1-28, the nucleic acid molecule of claim 29, the expression vector of claim 30, or the pharmaceutical composition of claim 31.

35. The method of claim 34, wherein the tumor is selected from malignant melanoma, non-small cell lung cancer, renal cell carcinoma, bladder cancer, gastric cancer, breast cancer, pancreatic cancer, and lung cancer.
